# EUROPÄISCHE PATENTSCHRIFT

(11) **EP 4 180 077 B1**
(45) Veröffentlichungstag und Bekanntmachung des Hinweises auf die Patenterteilung: **23.07.2025**
(21) Anmeldenummer: 22198781.1
(22) Anmeldetag: 29.09.2022
(51) Int. Cl.: A61M 16/06

(54) **VERBINDUNGSELEMENT FÜR EIN PATIENTENINTERFACE**
CONNECTING ELEMENT FOR A PATIENT INTERFACE
ELÉMENT DE CONNEXION POUR UNE INTERFACE PATIENT

(30) Priorität: 10.11.2021 DE 102021005565
(43) Veröffentlichungstag der Anmeldung: 17.05.2023
(73) Patentinhaber: Löwenstein Medical Technology S.A., 2557 Luxembourg (LU)
(72) Erfinder: Gruber, Jennifer, 21075 Hamburg (DE); Kamerling, Sarah, 49744 Geeste (DE)
(74) Vertreter: Löwenstein Medical IP

(56) Entgegenhaltungen:
- WO-A1-2011/060479
- WO-A1-2013/027174
- WO-A1-2017/017573
- WO-A1-2017/150990
- US-A1- 2017 049 984
- US-A1- 2020 269 001

## Beschreibung

Patienteninterfaces bzw. Atemmasken werden zur Beatmung, zur Unterstützung der Atmung oder als Schutzmaske gegen Aerosole aus festen oder flüssigen Partikeln eingesetzt. Atemmasken für die Beatmung oder Atemunterstützung bilden die Schnittstelle zwischen einem Anwender oder Patienten und einem Beatmungsgerät und müssen mit einem Verbindungselement am Kopf des Anwenders fixiert werden. Sowohl die Atemmasken als auch die Verbindungselemente müssen hohen Anforderungen an Stabilität und Sicherheit genügen und gleichzeitig einen ausreichenden Komfort und eine einfache Handhabung für den Anwender bieten. Zu unterscheiden sind Atemmasken mit Stirnstützen und Atemmasken ohne Stirnstützen.

Stirnstützenlose Atemmasken weisen üblicherweise Versteifungsvorrichtungen in den Verbindungselementen auf, um eine ausreichende Stabilität und einen guten Sitz der Atemmaske zu gewährleisten. Der versteifte Bereich ist dabei in der Regel zum Gesicht des Anwenders hin gepolstert, um den Tragekomfort zu erhöhen. Üblicherweise sind die Verbindungselemente komplex aufgebaut und mitunter nicht einfach anzuwenden. Solche Verbindungselemente werden beispielsweise in den Dokumenten WO 2017/017573 A1, WO 2017/150990 A1, US 2020/269001 A1, WO 2011/060479 A1 und WO 2013/027174 A1 gezeigt. Die US 2017/0049984 offenbart ein Verbindungselement für ein Patienteninterface nach dem Oberbegriff von Anspruch 1.

Es besteht somit Bedarf an Verbindungselementen, die einfach zu handhaben sind und einen stabilen, sicheren und komfortablen Sitz der Atemmaske sicherstellen.

Gelöst wird die Aufgabe durch ein Verbindungselement für ein Patienteninterface, die die kennzeichnenden Merkmale des Anspruchs 1 aufweist. Die Unteransprüche betreffen vorteilhafte Ausgestaltungen der Erfindung.

In manchen Ausführungsformen ist das Verbindungselement dadurch gekennzeichnet, dass das mindestens eine Hinterohrelement reversibel und/oder irreversibel an die Bügel koppelbar ist.

Das Verbindungselement ist dadurch gekennzeichnet, dass die Bügel einen Anfang und ein Ende umfassen, wobei benachbart zum Anfang ein Koppelsystem zur Kopplung des Bügels an das Patienteninterface eingerichtet und ausgebildet ist.

Das Verbindungselement ist dadurch gekennzeichnet, dass benachbart zum Ende ein Endabschnitt angeordnet ist, an den das Hinterohrelement koppelbar ist.

In manchen Ausführungsformen ist das Verbindungselement dadurch gekennzeichnet, dass die Bügel zumindest aus einem versteifenden und zugleich flexiblen Material und/oder Materialverbund hergestellt sind ausgewählt aus der Gruppe Kunststoffe, Metalle, Silikone und Schaumstoffe.

In manchen Ausführungsformen ist das Verbindungselement dadurch gekennzeichnet, dass die Bügel aus Polypropylen gefertigt sind.

In manchen Ausführungsformen ist das Verbindungselement dadurch gekennzeichnet, dass die Bügel eine Länge und eine Breite aufweisen, wobei das Verhältnis der Länge zur Breite mindestens 10:1, bevorzugt 20:1, besonders bevorzugt 25:1 oder mehr beträgt.

In manchen Ausführungsformen ist das Verbindungselement dadurch gekennzeichnet, dass die Bügel zusätzlich ein abpolsterndes Material mit elastischen und/oder viskoelastischen Eigenschaften aufweisen ausgewählt aus der Gruppe Schaumstoffe, Watte, Gewebe, Baumwolle, Wolle, Gummi, Neoprene und Gele.

In manchen Ausführungsformen ist das Verbindungselement dadurch gekennzeichnet, dass das Hinterohrelement aus einem elastischen Material gefertigt ist ausgewählt aus der Gruppe Silikone, Thermoplastischen Elastomere, Neoprene und Textilien.

In manchen Ausführungsformen ist das Verbindungselement dadurch gekennzeichnet, dass das Hinterohrelement aus Silikon gefertigt ist.

Das Verbindungselement ist dadurch gekennzeichnet, dass das Hinterohrelement mindestens ein Halteelement umfasst, das an den Endabschnitt des Bügels koppelbar ist.

Das Verbindungselement ist dadurch gekennzeichnet, dass das Halteelement als Hohlkörper mit einem Lumen ausgebildet ist, in das der Endabschnitt des Bügels zur Koppelung an mindestens einer Position platziert wird.

Das Verbindungselement ist dadurch gekennzeichnet, dass das Halteelement an beliebige Positionen des Endabschnitts verschiebbar gekoppelt wird und derart eine Längenverstellung des Verbindungselements gegeben ist.

In manchen Ausführungsformen ist das Verbindungselement dadurch gekennzeichnet, dass die Endabschnitte mindestens ein Ankerelement umfassen, um die Kopplung des Halteelements an den Bügel zu verankern.

In manchen Ausführungsformen ist das Verbindungselement dadurch gekennzeichnet, dass die Endabschnitte alternativ oder zusätzlich ein oder mehrere Fixierungselemente umfassen, um die Kopplung des Hinterohrelements an den Bügeln zu fixieren.

In manchen Ausführungsformen ist das Verbindungselement dadurch gekennzeichnet, dass die Fixierungselemente durch ein oder mehrere Verdickungen und/oder Einkerbungen des Endabschnitts der Bügel ausgebildet ist.

In manchen Ausführungsformen ist das Verbindungselement dadurch gekennzeichnet, dass das Hinterohrelement den Fixierungselementen entsprechende Gegenpart-Elemente aufweist, um die Fixierung des Hinterohrelements an den Bügel zu unterstützen.

In manchen Ausführungsformen ist das Verbindungselement dadurch gekennzeichnet, dass die Fixierungselemente in Form von Knöpfen, Pilzknöpfen, Haken, Stäben, Nieten oder Klettbändern mit Widerhaken und/oder Schlaufen ausgebildet sind und die Gegenpart-Elemente in Form von Knopflöchern, Löchern, Ösen, Schlaufen oder Klettbändern mit Widerhaken und/oder Schlaufen ausgebildet sind und die Fixierungselemente derart mit den Gegenpart-Elementen verbindbar sind, dass das Hinterohrelement an den Bügeln fixiert wird.

In manchen Ausführungsformen ist das Verbindungselement dadurch gekennzeichnet, dass die Fixierung des Hinterohrelements an den Bügeln eine stufenweise Längenverstellung des Verbindungselements zulässt.

In manchen Ausführungsformen ist das Verbindungselement dadurch gekennzeichnet, dass das Verbindungselement zusätzlich ein Kopfband umfasst, das an die Bügel koppelbar ist.

In manchen Ausführungsformen ist das Verbindungselement dadurch gekennzeichnet, dass die Bügel und das Hinterohrelement einstückig oder zweistückig ausgebildet sind.

In manchen Ausführungsformen ist das Verbindungselement dadurch gekennzeichnet, dass das Hinterohrelement eine Länge und eine Breite aufweist, wobei das Verhältnis der Länge zur Breite des Hinterohrelements 40 mindestens 1:1, bevorzugt 5:1 oder mehr beträgt.

In manchen Ausführungsformen ist das Verbindungselement dadurch gekennzeichnet, dass das Verbindungselement zwei Bügel und ein Hinterohrelement mit zwei Halteelementen umfasst, wobei das eine Halteelement an den einen Bügel gekoppelt wird und das zweite Halteelement an den zweiten Bügel gekoppelt wird.

In manchen Ausführungsformen ist das Verbindungselement dadurch gekennzeichnet, dass das eine Hinterohrelement in einem Anwendungszustand über den Hinterkopf eines Anwenders oder Patienten verläuft.

In manchen Ausführungsformen ist das Verbindungselement dadurch gekennzeichnet, dass das Verhältnis der Länge zur Breite des Hinterohrelements mindestens 5:1, bevorzugt 20:1, besonders bevorzugt 30:1 oder mehr beträgt.

In manchen Ausführungsformen ist das Verbindungselement dadurch gekennzeichnet, dass das Hinterohrelement in sich längenverstellbar ist.

In manchen Ausführungsformen ist das Verbindungselement dadurch gekennzeichnet, dass das Hinterohrelement mindestens eine Öse umfasst, die eingerichtet und ausgebildet ist, eine Längenverstellung zuzulassen.

Das Verbindungselement ist dadurch gekennzeichnet, dass das Verbindungselement zwei Bügel und zwei Hinterohrelemente mit jeweils einem Halteelement umfasst, wobei an jeden der beiden Bügel jeweils ein Hinterohrelement gekoppelt wird.

In manchen Ausführungsformen ist das Verbindungselement dadurch gekennzeichnet, dass das Verhältnis der Länge zur Breite der Hinterohrelemente jeweils weniger als 20:1, bevorzugt weniger als 10:1, besonders bevorzugt weniger als 5:1 beträgt.

In manchen Ausführungsformen ist das Verbindungselement dadurch gekennzeichnet, dass die Hinterohrelemente in einem Anwendungszustand hinter dem Ohr eines Anwenders oder Patienten verlaufen.

Die Aufgabe wird ferner gelöst durch ein System. Das System zur Beatmung und/oder Atemunterstützung umfasst mindestens ein Patienteninterface, das erfindungsgemäße Verbindungselement zur Verbindung mit dem Patienteninterface, ein Beatmungsgerät, mindestens eine Leitung, wobei das Patienteninterface mit dem Verbindungselement gekoppelt ist und über das Verbindungselement mit dem Kopf eines Patienten verbunden ist, wobei das Patienteninterface und das Beatmungsgerät über die Leitung gasleitend miteinander verbunden sind.

In den Figuren sind Ausführungsbeispiele des Verbindungselements dargestellt. Es zeigen:
**Fig. 1** beispielhaft eine Übersicht über das System mit einem Ausführungsbeispiel des Verbindungselements, das mit einem Patienteninterface verbunden ist.
**Fig. 2** eine perspektivische Draufsicht auf einen Teilbereich des Verbindungselements.
**Fig. 3** eine schematische Seitenansicht einer Ausführungsform des Verbindungselements in einem Anwendungszustand am Kopf eines Anwenders.
**Fig. 4** **und** **5** eine Seitenansicht des Bügels.
**Fig. 6A** das Verbindungselement nach Fig. 3 in einer Ansicht von oben und **Fig. 6B** eine Ansicht des Verbindungselements in einem Anwendungszustand am Kopf eines Anwenders von oben.
**Fig. 7 bis 9** unterschiedliche Ansichten des Verbindungselements aus Fig. 3, wobei **Fig. 7** eine perspektivische Ansicht des Verbindungselements, **Fig. 8** eine Ansicht des Verbindungselements von der Seite und **Fig. 9** eine Ansicht des Verbindungselements von vorne zeigen.
**Fig. 10** einen Teilbereich des Bügels in verschiedenen Ausführungsformen.
**Fig. 11 bis 14** unterschiedliche Ausführungsformen für ein Hinterohrelement des Verbindungselements aus Fig. 3.
**Fig. 15** eine schematische Seitenansicht einer alternativen Ausführungsform des erfindungsgemäßen Verbindungselements in einem Anwendungszustand am Kopf eines Anwenders.
**Fig. 16 bis 18** unterschiedliche Ansichten des erfindungsgemäßen Verbindungselements aus Fig. 15, wobei **Fig. 16** eine Ansicht des Verbindungselements von der Seite, **Fig. 17** eine perspektivische Ansicht des Verbindungselements, **Fig. 18A** eine Ansicht des Verbindungselements von vorne und Fig. **18B** eine Ansicht des Verbindungselements von oben zeigen.
**Fig. 19** ein Ausführungsbeispiel für das Hinterohrelement des erfindungsgemäßen Verbindungselements aus Fig. 15, wobei Fig. 19 A eine Ansicht von der Seite, Fig. 19 B eine Ansicht von vorne und Fig. 19 C eine perspektivische Ansicht zeigt.
**Fig. 20** alternative Ausführungsbeispiele für das Hinterohrelement des erfindungsgemäßen Verbindungselements aus Fig. 15 von der Seite.

Die in manchen Figuren dargestellten Koordinatensysteme dienen der Verdeutlichung der Ausrichtung der Ansichten. Dabei beschreiben die x-, y- und z-Achsen - soweit nicht anders beschrieben - in jeder Figur die gleiche Richtung. Eine mit einer x-Achse bezeichnete Richtung einer Figur entspricht also in der Regel der Richtung der x-Achse der anderen Figuren.

### Ausführungsbeispiele

In den nachfolgenden Ausführungsbeispielen ist ein Verbindungselement 10 gezeigt. Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt.

Das Verbindungselement 10 eignet sich beispielsweise für die Verwendung in einem System 100 zur Beatmung und/oder Atemunterstützung. Das System 100 umfasst ein Patienteninterface 90 und ein Beatmungsgerät 70, die über eine Leitung 80 gasleitend miteinander verbunden sind. Das Patienteninterface 90 ist über das Verbindungselement 10 mit dem Kopf 95 eines Patienten verbunden.

Das Verbindungselement 10 befindet sich in einem Anwendungszustand, wenn das Verbindungselement 10 zumindest teilweise zumindest bereichsweise an den Kopf 95 eines Patienten und/oder Anwenders angelegt ist.

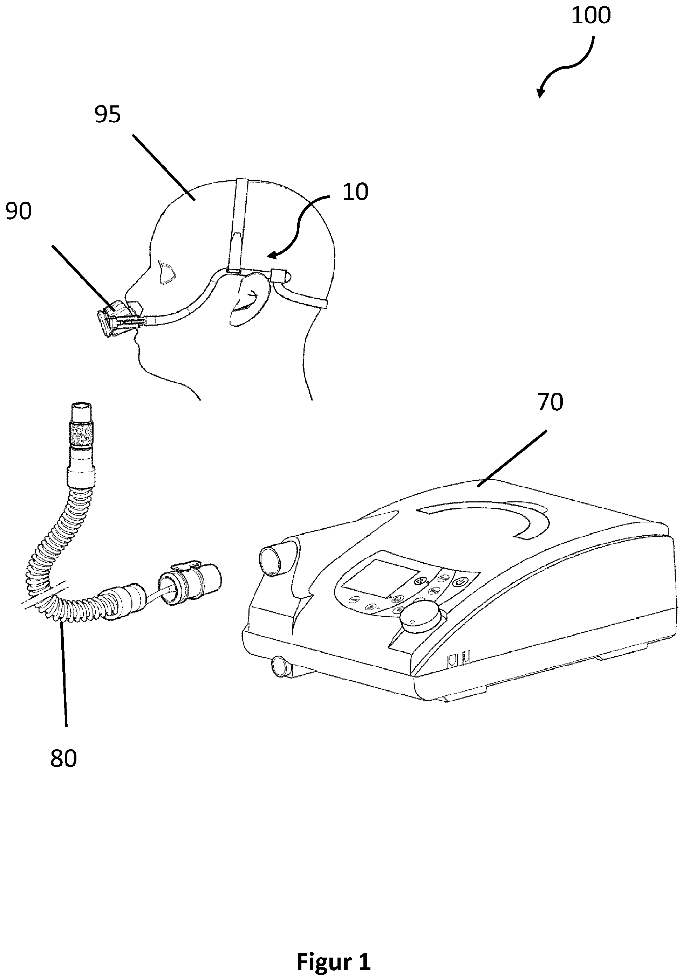

**Fig. 1** zeigt beispielhaft eine Übersicht über das System 100 mit einem Ausführungsbeispiel des Verbindungselements 10, das mit einem Patienteninterface 90 verbunden ist. Das Patienteninterface 90 kann über eine Leitung 80 mit einem Beatmungsgerät 70 gasleitend verbunden werden. In Fig. 1 sind Patenteninterface 90, Leitung 80 und Beatmungsgerät 70 nicht verbunden dargestellt. Eine gasleitende Verbindung kann hergestellt werden, indem die Leitung 80 beispielsweise über Anschlussstutzen an einem Ende mit dem Patienteninterface 90 und ein einem weiteren Ende mit dem Beatmungsgerät 70 verbunden wird (nicht gezeigt).

Als Patienteninterface 90 ist jegliches Peripheriegerät zu verstehen, welches zur Interaktion mit einem Lebewesen ausgebildet ist. Insbesondere ist das Patienteninterface 90 zu Therapie- und/oder Diagnosezwecken in Verbindung mit dem Beatmungsgerät 70 ausgebildet. Das Patienteninterface 90 kann als Atemmaske ausgebildet sein. Patienteninterface 90 und Atemmaske werden hierin als Synonyme verwendet.

Das Patienteninterface 90 kann als Nasenmaske ausgebildet sein und die Nase vollständig oder teilweise umschließen. Das Patienteninterface 90 kann auch als Nasenpolstermaske oder Nasenpillow-Maske ausgebildet sein. Nasenpillow-Masken finden beispielsweise in der CPAP- oder Bilevel-Therapie Anwendung. Das Patienteninterface 90 kann auch als Nasenbrille oder Sauerstoffbrille ausgebildet sein. Nasenbrillen finden beispielsweise in der Highflow-Therapie Anwendung. Nasenbrillen können Nasalprongs 92 aufweisen, die zumindest teilweise in die Nasenlöcher eingeführt werden können. Das Patienteninterface 90 kann auch als Full-Face-Maske oder Totalface-Maske ausgebildet sein, also Nase und Mund umschließen. Auch Trachealtuben bzw. -kanülen können als Patienteninterface 90 eingesetzt werden. Das Verbindungselement 10 eignet sich insbesondere für Nasenpillow-Masken und/oder für Highflow-Interfaces.

Das Patienteninterface 90 kann über ein Verbindungselement 10 an den Kopf 95 eines Anwenders oder Patienten angelegt bzw. befestigt werden. Anwender und Patient werden hierin gleichbedeutend verwendet. Wenn der Begriff Anwender oder Patient genannt wird, ist jegliches Individuum gemeint, dass eine Atemmaske 90 mit Verbindungselement 10 verwendet.

Unter einem Beatmungsgerät 70 sind sämtliche Geräte zu verstehen, die einen Anwender oder Patienten bei der natürlichen Atmung unterstützen und/oder die Beatmung eines Anwenders oder Patienten übernehmen und/oder einer Atemtherapie dienen und/oder anderweitig auf die Atmung eines Anwenders oder Patienten einwirken. Darunter fallen zum Beispiel, aber nicht ausschließlich, Atemtherapiegeräte, CPAP-, APAP- sowie BiLevel-Geräte, Highflow-Therapiegeräte, Narkose- bzw. Anästhesiegeräte, klinische, außerklinische oder Notfall-Beatmungsgeräte, Sauerstoff O₂-liefernde Geräte, Diagnosesysteme und Hustenmaschinen.

Patienteninterface 90 und Beatmungsgerät 70 sind bevorzugt über mindestens eine Leitung 80 miteinander verbunden. Die Leitung 80 ist als Gasleitung ausgebildet, die die einzelnen Komponenten des Systems 100 gasleitend miteinander verbindet. Die Leitung 80 ist bevorzugt flexibel und/oder drehbar ausgebildet. Die Leitung 80 kann beispielsweise als elastisches Rohr und/oder Schlauch und/oder Schlauchsystem ausgebildet sein.

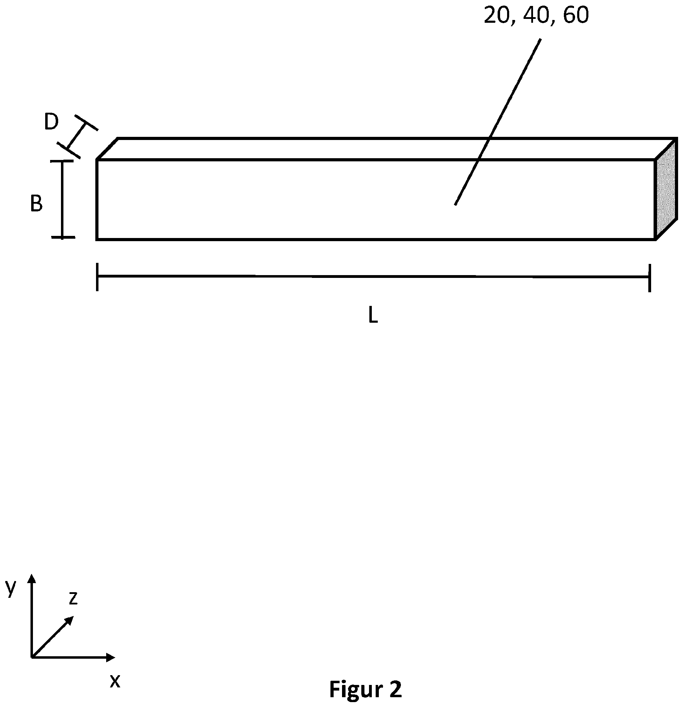

**Fig. 2** zeigt eine perspektivische Draufsicht auf einen Teilbereich des Verbindungselements 10. Das Verbindungselement 10 und Abschnitte des Verbindungselements 20, 40, 60 weisen eine Breite B, eine Dicke D und eine Länge L auf. Üblicherweise ist die Breite B größer als die Dicke D. Die Länge L ist üblicherweise größer als die Breite B. In einem Anwendungszustand, also wenn das Verbindungselement 10 an den hier nicht dargestellten Kopf 95 des Anwenders zumindest teilweise anliegt, ist das Verbindungselement 10 zumindest bereichsweise einseitig entlang der Breite B und entlang der Länge L dem Kopf 95 des Anwenders zugewandt.

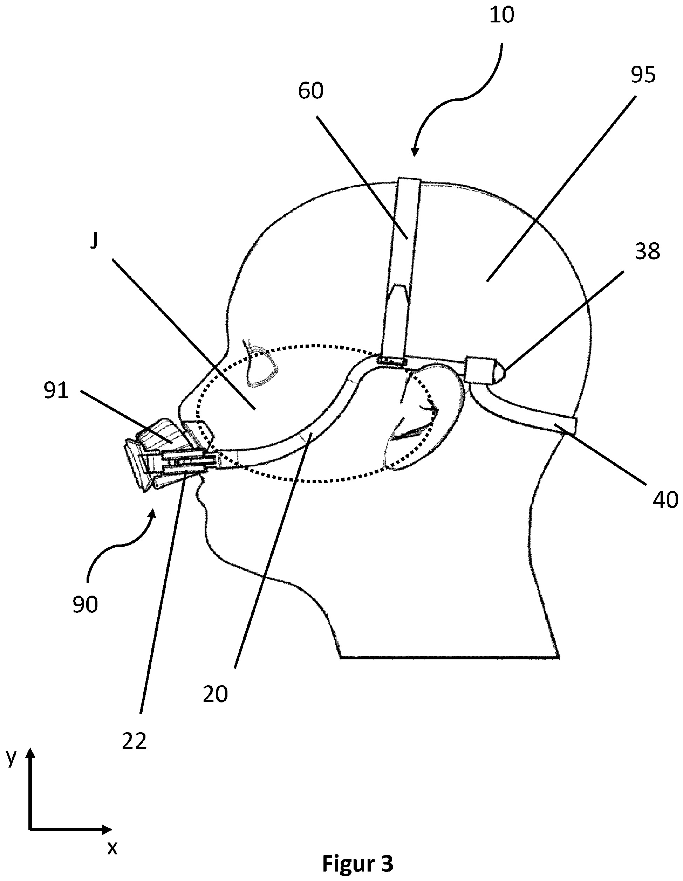

**Fig. 3** zeigt eine schematische Seitenansicht einer Ausführungsform des Verbindungselements 10 in einem Anwendungszustand am Kopf 95 eines Anwenders. Dieses Ausführungsbeispiel ist nicht erfindungsgemäß und dient lediglich der Veranschaulichung. Das Verbindungselement 10 umfasst mindestens einen Bügel 20 und mindestens ein Hinterohrelement 40. Zusätzlich kann das Verbindungselement mindestens ein Kopfband 60 umfassen.

Aus Fig. 3 wird ersichtlich, dass das Verbindungselement 10 in einem Anwendungszustand derart an dem Kopf 95 eines Patienten angeordnet ist, dass der Bügel 20 zumindest abschnittsweise zwischen der Nase und dem Ohr angeordnet ist. Hierbei befindet sich der Bügel 20 zumindest abschnittsweise in einer Jochbeinregion J.

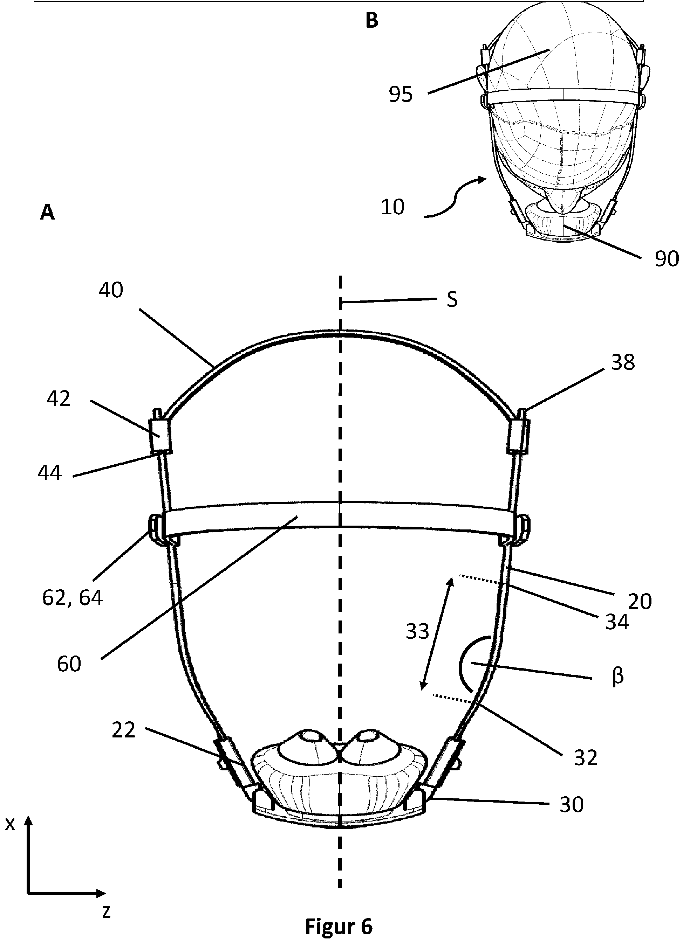

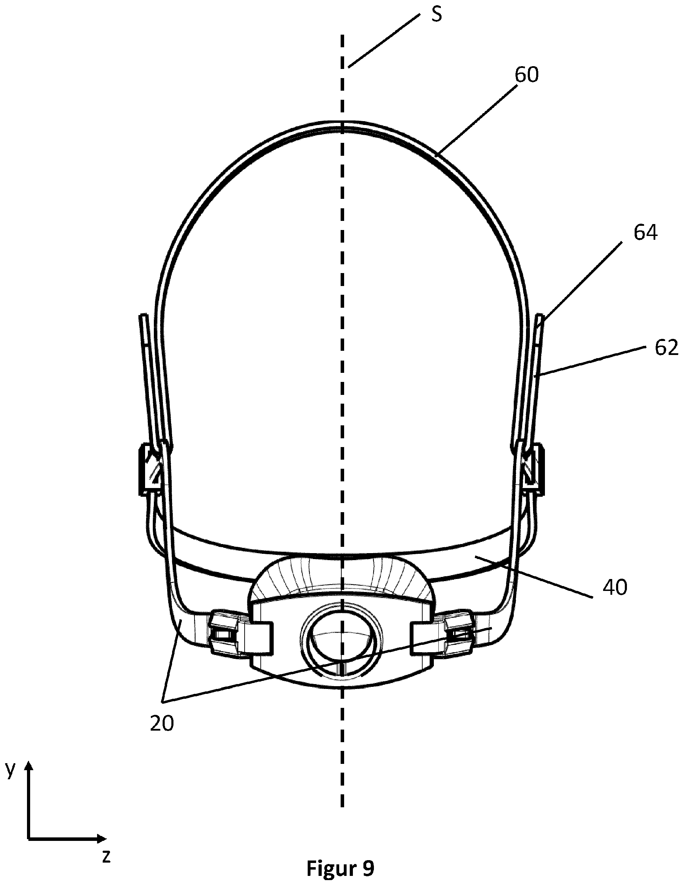

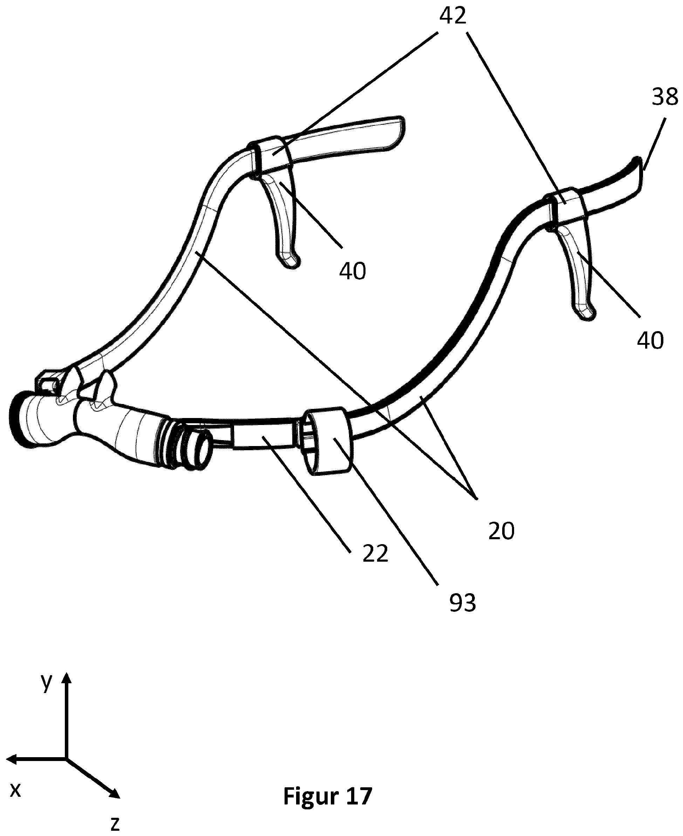

Das Verbindungselement 10 kann grundsätzlich spiegelsymmetrisch ausgebildet sein. Das Verbindungselement 10 kann daher eine gedachte Symmetrieebene S aufweisen (siehe Fig. 6A, 9), die parallel zu der durch die x- und y-Achse aufgespannten Ebene verläuft (siehe beispielsweise Fig. 3). Grundsätzlich spiegelsymmetrisch bedeutet, dass in der Regel keine perfekte Symmetrie vorliegt und die Spiegelbilder sich in ein oder mehreren Details unterscheiden können. Das Verbindungselement 10 kann zudem ein oder mehrere zusätzliche Elemente aufweisen, die unabhängig von der grundsätzlichen Symmetrie zusätzlich an einer Seite des Verbindungselements 10 angeordnet sein können. Als Beispiel sei hier eine Schlauchhalterung 93 genannt, die nur an einem der beiden Bügel 20 angeordnet sein kann (siehe beispielsweise Fig. 17). Im Folgenden wird in der Regel jeweils nur eine der spiegelsymmetrischen Hälften des Verbindungselements 10 beschrieben.

Das Verbindungselement 10 umfasst mindestens einen Bügel 20. In der Regel umfasst das Verbindungselement 10 zwei Bügel 20. Die Bügel 20 befinden sich in einem Anwendungszustand zum einen über Teilen der linken Gesichtshälfte und zum anderen über Teilen der rechten Gesichtshälfte (nicht gezeigt). Das Verbindungselement 10 kann auch mehr als zwei Bügel 20 umfassen.

Der Bügel 20 umfasst ein Koppelsystem 22. Über das Koppelsystem 22 kann das Verbindungselement 10 an das Patienteninterface 90 gekoppelt werden. Die Kopplung kann reversibel oder irreversibel erfolgen. Das Koppelsystem 22 kann einen Mechanismus zur Längenverstellung umfassen. Über das Koppelsystem 22 kann somit die Länge L des Verbindungselements 10 angepasst werden. Die Kopplung kann beweglich oder unbeweglich sein.

Das Patienteninterface 90 kann als Nasenpillow-Maske ausgebildet sein. Nasenpillow-Masken können beispielsweise für CPAP- oder Bilevel-Therapien verwendet werden. Hierfür decken die Nasenpillow-Masken mit einem Nasenpillow 91 die Nasenlöcher und/oder die Umgebung der Nasenlöcher gasdichtend ab. Das Nasenpillow 91 kann zumindest teilweise in die Nasenlöcher eingeführt werden.

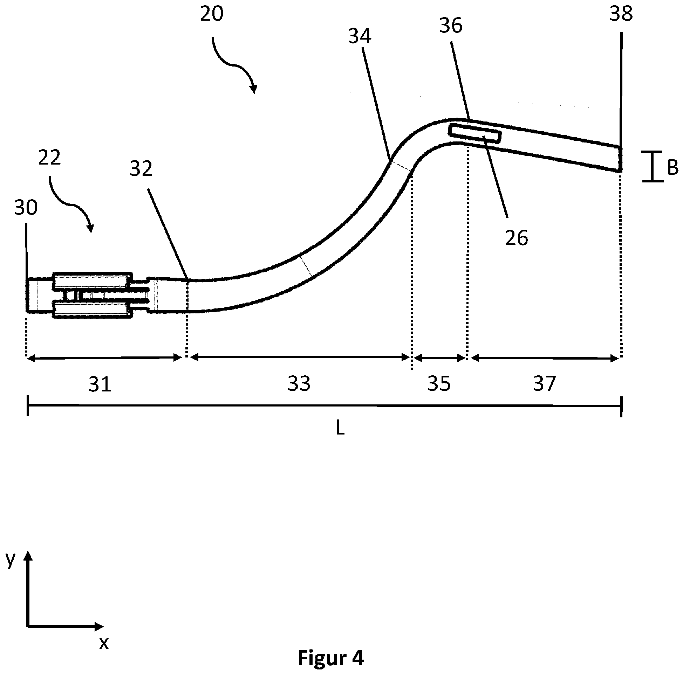

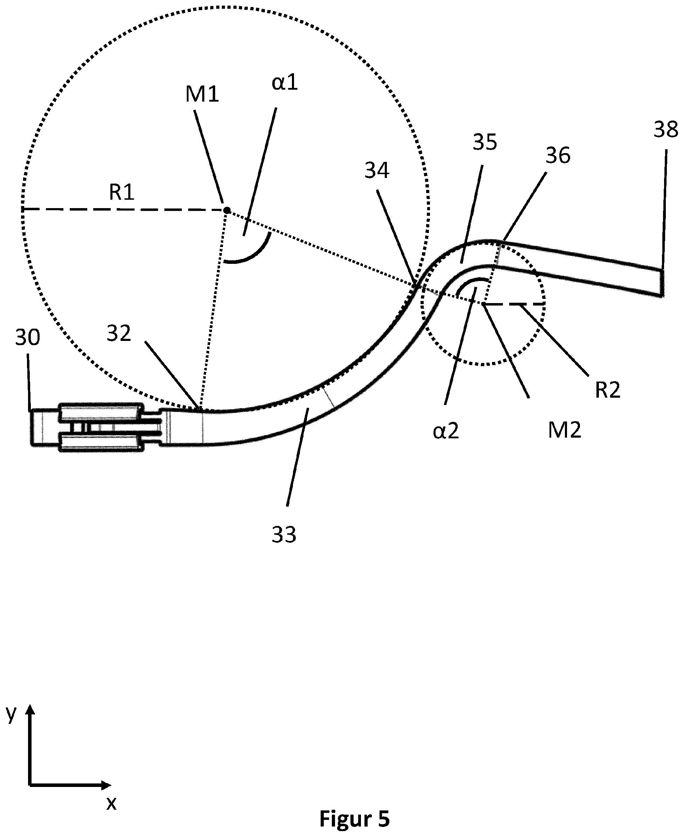

**Fig. 4** **und** **Fig. 5** zeigen eine Seitenansicht des Bügels 20. Der Bügel 20 umfasst einen Anfang 30 und einen Ende 38. Anfang 30 und Ende 38 liegen entlang der Länge L des Bügels 20 an entgegengesetzten Enden des Bügels 20. Der Bügel 20 endet an der einen Seite am Anfang 30 und an der anderen Seite am Ende 38. In einem Anwendungszustand kann der Anfang 30 benachbart zum Patienteninterface 90 angeordnet sein und das Ende 38 an der gegenüberliegenden Seite. Das Ende 38 kann in einem Anwendungszustand am und/oder hinter dem Ohr angeordnet sein (siehe Fig. 3).

Der Bügel 20 erstreckt sich der Länge L nach vom Anfang 30 zum Ende 38. Die Länge L des Bügels 20 ist wesentlich größer als die Breite B des Bügels 20. Das Verhältnis der Länge L zur Breite B des Bügels 20 kann in einem Bereich von 10:1 bis 40:1 liegen. Beispielsweise beträgt das Verhältnis der Länge L zur Breite B des Bügels 20 mindestens 10:1. In manchen Ausführungsformen beträgt das Verhältnis der Länge L zur Breite B des Bügels 20 mindestens 20:1. In einer bevorzugten Ausführungsform beträgt das Verhältnis der Länge L zur Breite B des Bügels 20 beispielsweise 25:1 oder mehr. Der Bügel 20 weist eine besonders langgestreckte, schmale Form auf.

Die Breite B des Bügels 20 kann konstant oder variabel ausgebildet sein. In einer beispielhaften Ausführungsform kann die Breite B des Bügels 20 im Wesentlichen konstant ausgebildet sein und zum Anfang 30 und/oder zum Ende 38 hin in der Breite B zu- oder abnehmen.

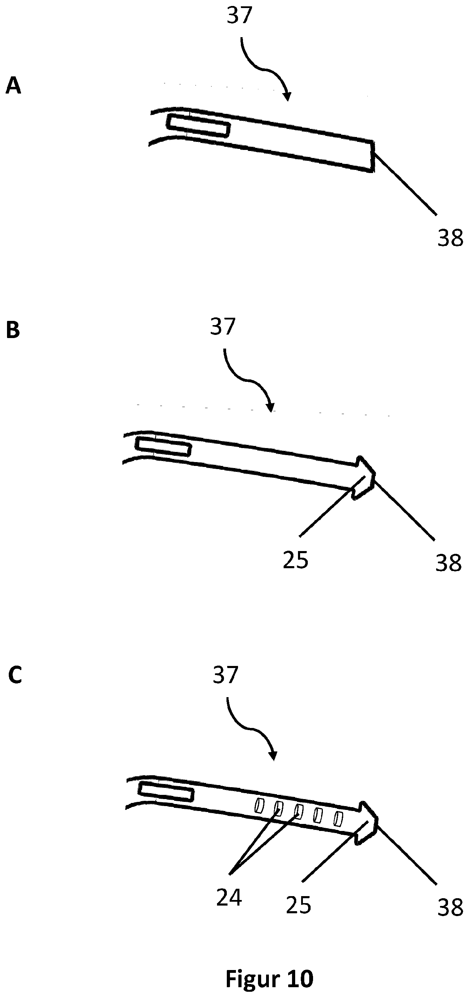

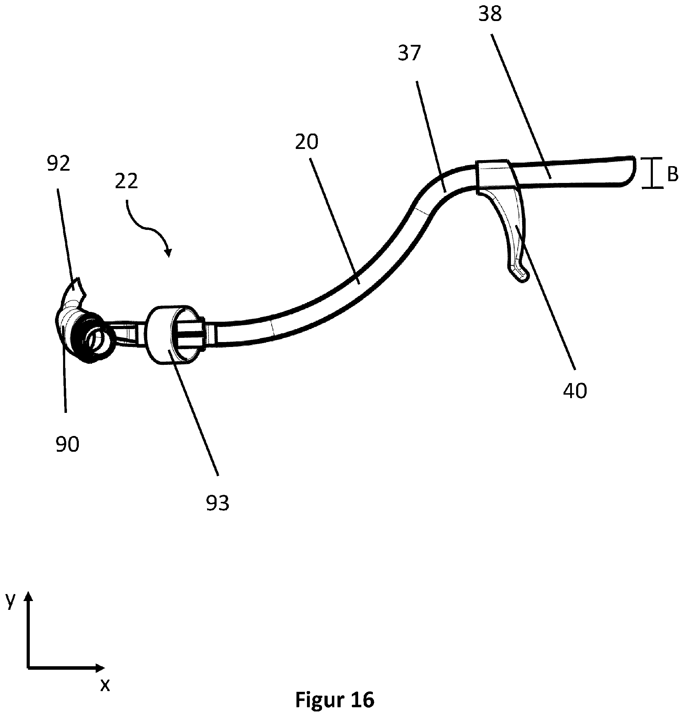

In einer bevorzugten Ausführungsform nimmt die Breite B zum Ende 38 hin zu (siehe beispielsweise Fig. 10B, Fig. 16). Die Breite B kann zum Anfang 30 hin zu- oder abnehmen. In einer bevorzugten Ausführungsform nimmt die Breite B zum Anfang 30 hin zu (Fig. 4).

Die Breite B des Bügels 20 kann in einem Bereich zwischen 1 bis 20 mm liegen. Bevorzugt liegt die Breite B des Bügels 20 zwischen 3 und 10 mm. In einem konkreten Ausführungsbeispiel beträgt die Breite B des Bügel 20 im Wesentlichen 7 mm.

Die Länge L des Bügels 20 kann in einem Bereich zwischen 50 bis 250 mm liegen. Bevorzugt liegt die Länge L des Bügels 20 zwischen 150 und 220 mm. In einem konkreten Ausführungsbeispiel beträgt die Länge L des Bügel 20 beispielhaft 200 mm.

Aus Fig. 4 wird ersichtlich, dass der Bügel 20 zumindest abschnittsweise gerade und/oder gebogen ausgebildet sein kann. Fig. 4 und Fig. 5 zeigen, dass der Bügel 20 mehrere Abschnitte 31, 33, 35, 37 umfasst, die unterschiedlich geformt sein können. Der Bügel 20 umfasst mehrere Wendepunkte 30, 32, 34, 36, 38, zwischen denen Abschnitte 31, 33, 35, 37 angeordnet sind.

Der Anfang 30 des Bügels 20 kann im Schnittpunkt der x- und der y-Achse liegen. Der Anfang 30 definiert ein erstes äußeres Ende des Bügels 20, nämlich jenes, das in einem Anwendungszustand benachbart zum Patienteninterface 90 und zum Koppelsystem 22 ist. Das Ende 38 des Bügels 20 definiert ein zweites äußeres Ende des Bügels 20, nämlich jenes, welches in einem Anwendungszustand am und/oder hinter dem Ohr angeordnet ist. Der Bügel 20 kann zwischen dem Anfang 30 und dem Ende 38 im x/y-Koordinatensystem gerade und/oder gebogen oder gekrümmt verlaufen. Der Bügel 20 erstreckt sich im x/y-Koordinatensystem bevorzugt so, dass das Ende 38 in y-Richtung oberhalb des Anfangs 30 liegt.

Zwischen dem Anfang 30 und dem Ende 38 liegen mindestens drei Wendepunkte 32, 34, 36. In dem in Fig. 4/5 dargestellten konkreten Ausführungsbeispiel liegen zwischen dem Anfang 30 und dem Ende 38 beispielsweise drei Wendepunkte 32, 34, 36. In alternativen Ausführungsformen können auch weniger als drei oder mehr als drei Wendepunkte ausgebildet sein.

Zwischen dem Anfang 30 und den Wendepunkten 32, 34, 36 und dem Ende 38 liegen mindestens vier Abschnitte 31, 33, 35, 37. In dem in Fig. 4/5 dargestellten konkreten Ausführungsbeispiel liegen zwischen dem Anfang 30 und dem Ende 38 beispielsweise vier Abschnitte 31, 33, 35, 37. In alternativen Ausführungsformen können auch weniger als vier oder mehr als vier Abschnitte ausgebildet sein. Die Abschnitte 31, 33, 35, 37 können gerade und/oder gebogen oder gekrümmt ausgebildet sein.

Zwischen dem Anfang 30 und dem Wendepunkt 32 kann ein Abschnitt 31 des Bügels 20 angeordnet sein. Abschnitt 31 bezeichnet denjenigen Bügelabschnitt, der zwischen dem Anfang 30 und dem Wendepunkt 32 liegt. Der Abschnitt 31 kann gerade oder gebogen oder gekrümmt sein. In dem konkreten Ausführungsbeispiel nach Fig. 4 ist der Abschnitt 31 in Richtung der x-Achse im Wesentlichen gerade ausgebildet. In dem Abschnitt 31 kann das Koppelsystem 22 angeordnet sein. Im Abschnitt 31 kann ein Mechanismus zur Längenverstellung angeordnet sein.

Zwischen dem Wendepunkt 32 und dem Wendepunkt 34 kann ein weiterer Abschnitt 33 des Bügels 20 angeordnet sein. Abschnitt 33 bezeichnet denjenigen Bügelabschnitt, der zwischen dem Wendepunkt 32 und dem Wendepunkt 34 liegt. Der Abschnitt 33 kann gerade oder gebogen oder gekrümmt sein. In dem konkreten Ausführungsbeispiel nach Fig. 4/5 ist der Abschnitt 31 im x/y-Koordinatensystem gebogen ausgebildet. Der Wendepunkt 34 liegt in y-Richtung oberhalb des Wendepunktes 32.

Zwischen dem Wendepunkt 34 und dem Wendepunkt 36 kann ein weiterer Abschnitt 35 des Bügels 20 angeordnet sein. Abschnitt 35 bezeichnet denjenigen Bügelabschnitt, der zwischen dem Wendepunkt 34 und dem Wendepunkt 36 liegt. Der Abschnitt 35 kann gerade oder gebogen oder gekrümmt sein. In dem konkreten Ausführungsbeispiel nach Fig. 4/5 ist der Abschnitt 35 im x/y-Koordinatensystem gebogen ausgebildet. Der Wendepunkt 36 liegt in y-Richtung oberhalb des Wendepunktes 34.

**Fig. 5** verdeutlicht die Biegung der Abschnitte 33 und 35 des konkreten Ausführungsbeispiels nach Fig. 4. Abschnitt 33 verläuft zwischen den Wendepunkten 32 und 34 gebogen. Die Biegung entspricht einem Kreisbogen eines gedachten Kreises oder Ovals mit einem Radius R1. Der Radius R1 kann beispielsweise in einem Bereich zwischen 60 mm und 75 mm liegen. Verbindet man die Wendepunkte 32 und 34 mit einem Mittelpunkt M1 des Kreises oder Ovals, so erhält man einen Kreisausschnitt mit einem Mittelpunktswinkel α1. Der Mittelpunktswinkel α1 kann in einem Bereich zwischen 30° und 80° liegen. Beispielsweise liegt der Mittelpunktswinkel α1 in einem Bereich zwischen 60° und 70°.

Abschnitt 35 verläuft zwischen den Wendepunkten 34 und 36 gebogen. Die Biegung entspricht einem Kreisbogen eines gedachten Kreises oder Ovals mit einem Radius R2. Der Radius R2 kann beispielsweise in einem Bereich zwischen 10 mm und 20 mm liegen. Verbindet man die Wendepunkte 34 und 36 mit einem Mittelpunkt M2 des Kreises oder Ovals, so erhält man einen Kreisausschnitt mit einem Mittelpunktswinkel α2. Der Mittelpunktswinkel α2 kann in einem Bereich zwischen 30° und 80° liegen. Beispielsweise liegt der Mittelpunktswinkel α2 in einem Bereich zwischen 60° und 75°.

Die Mittelpunktswinkel α1 und α2 können gleich groß sein oder in ihrer Größe voneinander abweichen.

Die Biegung oder Krümmung des Abschnitts 35 kann beispielsweise entgegengesetzt zu der Biegung oder Krümmung des Abschnitts 33 ausgebildet sein. So kann sich beispielsweise ein konvex gebogener Bügelabschnitt 35 an einen konkav gebogenen Bügelabschnitt 33 anschließen und vice versa.

Der Bügel 20 kann in alternativen Ausführungsformen statt den in Fig. 4/5 dargestellten bogenförmigen Krümmungen auch aus geraden Abschnitten 31, 33, 35, 37 ausgebildet sein, die zueinander derart abgeknickt vorliegen, dass das Ende 38 in y-Richtung oberhalb des Anfangs 30 liegt (nicht gezeigt).

Zwischen dem Wendepunkt 36 und dem Ende 38 ist ein Endabschnitt 37 des Bügels 20 angeordnet. Endabschnitt 37 kann denjenigen Bügelabschnitt bezeichnen, der zwischen dem Wendepunkt 36 und dem Ende 38 liegt. Der Endabschnitt 37 liegt benachbart zum Ende 38 und bezeichnet den letzten Abschnitt des Bügels 20.

Der Endabschnitt 37 kann gerade und/oder gebogen oder gekrümmt sein. In dem konkreten Ausführungsbeispiel nach Fig. 4 ist der Endabschnitt 37 in Richtung der x-Achse im Wesentlichen gerade ausgebildet.

In manchen Ausführungsformen kann der Endabschnitt 37 wesentlich länger als in Fig. 4/5 dargestellt ausgebildet sein. Die Länge L des Endabschnitts 37 kann bis zu 80% der Gesamtlänge L des Bügels 20 ausmachen. Bevorzugt macht die Länge L des Endabschnitts 37 weniger als 50 % der Gesamtlänge L des Bügels 20 aus. In einem konkreten Ausführungsbeispiel macht die Länge L des Endabschnitts 37 20 % bis 25 % der Gesamtlänge L des Bügels 20 aus. Ein kurzer Endabschnitt 37 wirkt sich positiv auf den Tragekomfort für den Anwender aus, beispielsweise wenn der Anwender den Kopf im Liegen, zum Beispiel beim Schlafen, ablegt.

In manchen Ausführungsformen ist denkbar, dass ein langgestreckt ausgebildeter Endabschnitt 37 gerade und gekrümmt oder gebogen ausgebildet sein kann. Es ist beispielsweise denkbar, dass der Endabschnitt 37 des Bügels 20 zumindest abschnittsweise derart gekrümmt vorliegt, dass er in einem Anwendungszustand das Ohr zumindest teilweise umläuft (nicht gezeigt).

In dem in Fig. 4/5 dargestellten konkreten Ausführungsbeispiel liegen zwischen dem Anfang 30 und dem Ende 38 beispielsweise vier Abschnitte 31, 33, 35, 37. In alternativen Ausführungsformen können auch weniger als vier oder mehr als vier Abschnitte ausgebildet sein.

Der Endabschnitt 37 ist zumindest abschnittsweise eingerichtet und ausgebildet eine Kopplung mit dem Hinterohrelement 40 einzugehen. Über den Endabschnitt 37 kann das Hinterohrelement 40 mit dem Bügel 20 verbunden werden.

Der Bügel 20 kann darüber hinaus in manchen Ausführungsformen mindestens ein Kopplungselement 26 umfassen. Das Kopplungselement 26 kann beispielsweise und bevorzugt in Form eines Durchbruchs im Bügel 20 ausgebildet sein. Das Kopplungselement 26 kann auch als zusätzliches Element ausgebildet sein, beispielsweise als Schlaufe oder Öse, das an den Bügel 20 angeformt ist (nicht gezeigt).

Das mindestens eine Kopplungselement 26 ist eingerichtet und ausgebildet, das Kopfband 60 als zusätzliches Verbindungselement an den Bügel 20 zu koppeln. Das Kopplungselement 26 kann an sämtlichen Abschnitten 31, 33, 35, 37 des Bügels 20 angeordnet sein. Bevorzugt ist das Kopplungselement 26 in Abschnitt 35 und/oder im Endabschnitt 37 angeordnet.

Das Kopplungselement 26 kann in einem konkreten Ausführungsbeispiel durch eine Aussparung im Material des Bügels 20 ausgebildet sein (siehe Fig. 4). Das Kopfband 60 kann durch das Kopplungselement 26 hindurchgeführt werden. Zu diesem Zweck ist das Kopplungselement 26 bevorzugt formkomplementär zum Kopfband 60 ausgebildet. Beispielsweise ist das Kopplungselement 26 minimal breiter als das Kopfband 60 breit ist und minimal höher als das Kopfband 60 dick ist.

Der Bügel 20 ist bevorzugt zumindest aus einem festen, versteifenden Material ausgebildet, das dennoch eine gewisse Flexibilität aufweist. Die Bügel 20 sollte eine gewisse Steifigkeit bei gleichzeitiger Flexibilität aufweisen, um dem Verbindungselement 10 insgesamt eine ausreichende Stabilität zu verleihen und trotzdem eine flexible Anlage am Kopf 95 zu ermöglichen.

Der Bügel 20 kann in einer einfachen Ausführungsform aus nur einem Material gefertigt sein, das eine Schicht bildet. In manchen Ausführungsformen ist es auch möglich, dass der Bügel 20 aus mehreren Schichten aufgebaut ist.

Der Bügel 20 kann aus Kunststoff, Metall, Silikon, Schaumstoff und/oder einem Materialverbund ausgebildet sein. Das Grundmaterial des Bügels 20 kann beispielsweise Hartplastik und/oder Hartkunststoff sein. Beispielsweise ist der Bügel 20 aus Polypropylen gefertigt.

Der Bügel 20 kann in einem Grundmaterial zumindest bereichsweise zusätzliche verstärkende und/oder flexible und/oder verformbare Elemente aufweisen. Beispielsweise kann in ein Grundmaterial aus Kunststoff zusätzlich beispielsweise Draht, anorganische und/oder organische Verstärkungsfasern, Kurz- und/oder Langfasern, Stäbe, Streifen, Gewebe, Geflechte, Gitter und/oder Strukturmatten angeordnet sein.

In manchen Ausführungsformen kann der Bügel 20 als verformbares Element ausgebildet sein, das individuell an die Kopfform angepasst werden kann. Beispielsweise kann der Bügel 20 aus mindestens einem Draht aus einer Knetlegierung ausgebildet sein, beispielsweise aus einer AluminiumKnetlegierung. Andere Materialien mit verformbaren Eigenschaften sind auch denkbar, beispielsweise Kunststoffe wie Thermoplaste oder ähnliche. In einer alternativen Ausführungsform kann der Bügel 20 aus einem Materialverbund ausgebildet sein, vorzugsweise aus einem Hartkunststoff und Silikon und/oder aus einem Hartkunststoff und einem thermoplastischen Elastomer (TPE).

Der Bügel 20 ist nicht auf diese Ausführungsformen beschränkt. Jegliche Materialien, die verstärkende und/oder versteifende und/oder flexible Eigenschaften aufweisen sind denkbar. Der Bügel 20 kann in einer einfachen Ausführungsform aus nur einem der oben genannten Materialien oder Materialverbunde gefertigt sein.

In manchen Ausführungsformen kann der Bügel 20 ergänzend zumindest bereichsweise von einem Textil umgeben sein. Das Textil kann den Tragekomfort des Bügels 20 erhöhen. Denkbare Textilien können Baumwolle, Seide und/oder aus synthetischen Fasern wie beispielsweise aus Polyester, Polyurethan, Neopren, Spandex und/oder Nylon sein. Mischgewebe sind auch denkbar. Denkbar sind auch andere Stoffe, die in Bezug auf Biokompatibilität, Komfort, Stabilität, Elastizität und Kosten geeignet erscheinen.

Alternativ oder zusätzlich kann der Bügel 20 zumindest bereichsweise ein abpolsterndes Material aufweisen. Das abpolsternde Material kann beispielsweise aus Schaumstoff, Watte, Gewebe, Baumwolle, Wolle, Gummi, Neopren, Gel und/oder anderen elastischen und/oder viskoelastischen Materialien gefertigt sein. Das abpolsternde Material kann den Tragekomfort erhöhen und/oder das Tragen einer Brille angenehmer machen.

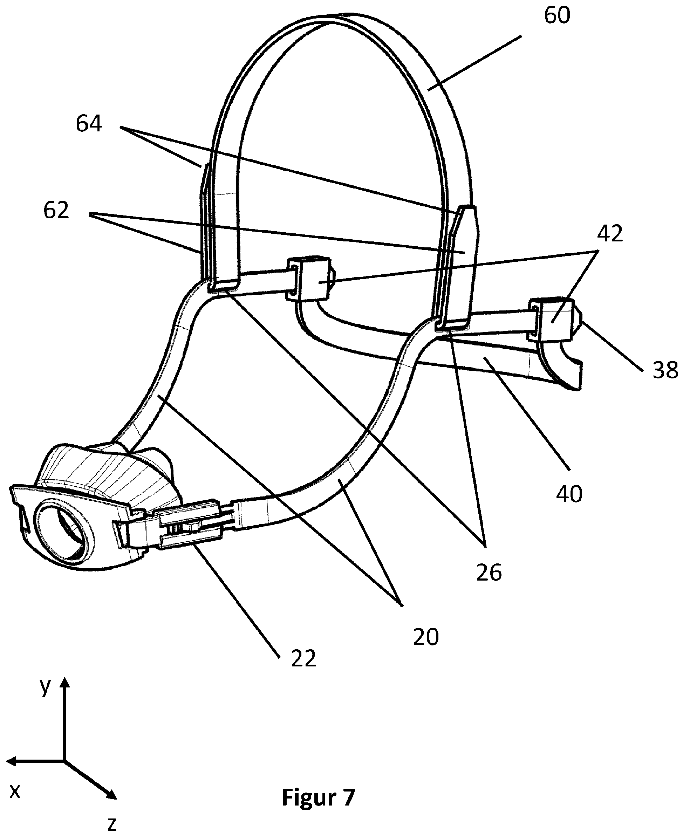

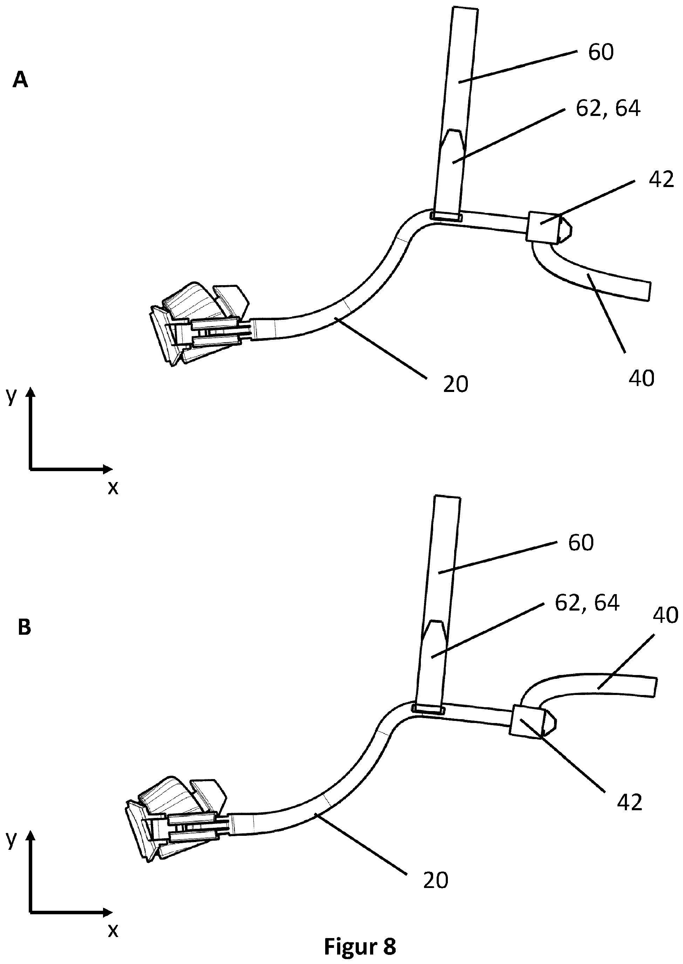

**Fig. 6 bis 9** zeigen Ansichten des Verbindungselements 10 aus Fig. 3, wobei **Fig. 6A** eine Ansicht des Verbindungselements 10 von oben, **Fig. 6B** eine Ansicht des Verbindungselements 10 in einem Anwendungszustand am Kopf 95 eines Anwenders von oben, **Fig. 7** eine perspektivische Ansicht des Verbindungselements 10, **Fig. 8** eine Ansicht des Verbindungselements 10 von der Seite und **Fig. 9** eine Ansicht des Verbindungselements 10 von vorne zeigen.

Die Bügel 20 können im x/z-Koordinatensystem gerade und/oder gebogen oder gekrümmt ausgebildet sein. Bevorzugt befindet sich innerhalb des x/z-Koordinatensystems mindestens eine Krümmung und/oder Biegung des Bügels 20. Durch die Biegung oder Krümmung der Bügel 20 im x/z-Koordinatensystem können die Bügel 20 vom Patienteninterface 90 zu den Ohren verlaufen und an die Kopfform des Anwenders und/oder Patienten angepasst sein. Durch die Biegung oder Krümmung der Bügel 20 im x/z-Koordinatensystem kann das Verbindungselement 10 am Patienteninterface 90 schmaler ausgebildet sein als an den Bereichen, die im Anwendungszustand am Kopf 95 des Anwenders anliegen (siehe Fig. 6B). Das Ende 38 des Bügels liegt durch die Biegung oder Krümmung in z-Richtung über und/oder unter dem Anfang des Bügels 30.

Die Abschnitte 31, 33, 35, 37 können im x/z-Koordinatensystem gerade und/oder gebogen oder gekrümmt ausgebildet sein. Es können alle Abschnitte 31, 33, 35, 37 im x/z-Koordinatensystem gebogen oder gekrümmt ausgebildet sein. In manchen Ausführungsformen können auch nur einige oder nur einer der Abschnitte 31, 33, 35, 37 im x/z-Koordinatensystem gebogen oder gekrümmt ausgebildet sein.

In dem konkreten Ausführungsbeispiel nach Fig. 6 ist mindestens ein Abschnitt des Bügels 20 im x/z-Koordinatensystem gebogen oder gekrümmt ausgebildet sein. Beispielhaft ist der Abschnitt 31 im x/z-Koordinatensystem über einen Winkel β gekrümmt ausgebildet. Sodann liegt der Wendepunkt 32 in z-Richtung oberhalb des Wendepunktes 34. Der Winkel β liegt in einem Bereich von 120° bis 180°. Beispielsweise liegt der Winkel β in einem Bereich zwischen 130° und 170°.

Der Bügel 20 ist zwischen dem Anfang 30 und dem Ende 38 bevorzugt in zwei Ebenen, nämlich sowohl im x/y-Koordinatensystem als auch im x/z-Koordinatensystem, zumindest abschnittsweise gebogen oder gekrümmt.

Das Hinterohrelement weist eine Länge L und eine Breite B auf. In einem einfachen Ausführungsbeispiel kann die Länge L gleich der Breite B sein. Das Verhältnis der Länge L zur Breite B des Hinterohrelements 40 kann 1:1 betragen (nicht gezeigt). In bevorzugten Ausführungsformen kann das Verhältnis der Länge L zur Breite B des Hinterohrelements 40 bevorzugt 5:1 oder mehr betragen.

Aus Fig. 6 und Fig. 7 ist ersichtlich, dass das Hinterohrelement 40 in diesem Ausführungsbeispiel derart lang eingerichtet und ausgebildet ist, dass es vom Ende 38 des einen Bügels 20 zum Ende 38 des zweiten Bügels 20 verlaufen kann. In einem Anwendungszustand ist das Hinterohrelement 40 am Hinterkopf des Anwenders angeordnet. In dem Ausführungsbeispiel gemäß Fig. 3 ff ist lediglich ein einziges Hinterohrelement 40 an den Bügeln 20 angeordnet.

Das Hinterohrelement 40 weist beispielsweise eine langgestreckte Form mit zwei Enden 44 auf. Die Enden 44 können derart geformt sein, dass sie ein oder mehrere Halteelemente 42 ausbilden. Über die beispielsweise zwei Halteelemente 42 kann das Hinterohrelement 40 an die Enden 38 der beiden Bügel 20 gekoppelt werden. Diese Kopplung kann irreversibel oder reversibel sein. Diese Kopplung kann beweglich oder unbeweglich eingerichtet sein. Über die Kopplung von Hinterohrelement 40 und Bügel 20 kann eine Längenverstellung erfolgen. Je nachdem an welcher Position die Halteelemente 42 auf dem Bügel 20 platziert werden, kann das Verbindungselement 10 weiter oder enger eingestellt werden (siehe auch weiter unten hierin).

Das Verbindungselement 10 kann in dem in den Figuren 3 und 5 bis 8 dargestellten Ausführungsbeispiel zusätzlich zum Bügel 20 und zum Hinterohrelement 40 ein weiteres Element in Form mindestens eines Kopfbands 60 umfassen.

Das Kopfband 60 kann an dem Bügel 20 befestigt werden. Die Befestigung kann lösbar und/oder nichtlösbar eingerichtet sein. Bevorzugt kann das Kopfband 60 lösbar an dem Bügel 20 befestigt werden. Das Kopfband 60 kann durch die Durchbrüche 26 der Bügel 20 hindurchführen, um das Kopfband 60 an die Bügel 20 zu koppeln.

Das Kopfband 60 kann eine langgestreckte schmale Form aufweisen. Die Länge L des Kopfbandes 60 ist wesentlich größer als die Breite B des Kopfbandes 60. Das Verhältnis der Länge L zur Breite B des Kopfbandes 60 kann in einem Bereich von 10:1 bis 50:1 liegen. Beispielsweise beträgt das Verhältnis der Länge L zur Breite B des Kopfbandes 60 mindestens 10:1. In manchen Ausführungsformen beträgt das Verhältnis der Länge L zur Breite B des Kopfbandes 60 mindestens 20:1. In einer bevorzugten Ausführungsform beträgt das Verhältnis der Länge L zur Breite B des Kopfbandes 60 28:1 oder mehr. Das Kopfband 60 weist bevorzugt eine langgestreckte, schmale Form auf.

Die Breite B des Kopfbandes 60 kann konstant oder variierend ausgebildet sein. In einer beispielhaften Ausführungsform kann die Breite B des Kopfbandes 60 konstant ausgebildet sein. Die Breite B des Kopfbandes 60 kann in einem Bereich zwischen 1 bis 50 mm liegen, bevorzugt in einem Bereich zwischen 3 bis 30 mm. Besonders bevorzugt liegt die Breite B des Kopfbandes 60 zwischen 5 und 20 mm. In einem konkreten Ausführungsform beträgt die Breite B des Kopfbandes 60 im Wesentlichen 12 mm.

Die Länge L des Kopfbandes 60 kann in einem Bereich zwischen 150 bis 500 mm liegen. Bevorzugt liegt die Länge L des Kopfbandes 60 zwischen 300 und 500 mm. In einem konkreten Ausführungsbeispiel beträgt die Länge L des Kopfbandes 60 beispielhaft 340 mm.

Das Kopfband 60 kann aus einem Textil gefertigt sein. Das Material des Kopfbandes 60 ist bevorzugt weich und flexibel. Das Kopfband 60 ist besonders bevorzugt dehnbar ausgebildet. Denkbare Textilien können Baumwolle, Seide und/oder aus synthetischen Fasern wie beispielsweise aus Polyester, Polyurethan, Neopren, Spandex und/oder Nylon sein. Mischgewebe sind auch denkbar. Denkbar sind auch andere Stoffe, die in Bezug auf Biokompatibilität, Komfort, Stabilität, Elastizität und Kosten geeignet erscheinen.

In einer einfachen Ausführungsform ist das Kopfband 60 lediglich aus einem Textil gefertigt und ist eingerichtet und ausgebildet, dem Verbindungselement 10 zusätzlichen Halt und Stabilität zu verleihen. Alternativ oder zusätzlich kann das Kopfband 60 auch verstärkende und/oder abpolsternde Materialien aufweisen. Dies kann von Vorteil sein, um den Tragekomfort zu erhöhen und/oder das Anlegen an den Kopf 95 zu erleichtern. Beispielsweise ist es denkbar, dass das Kopfband 60 verstärkende Strukturen beinhaltet, die das Kopfband 60 in einer für die Kopfform optimalen Form bereitstellt.

Das Kopfband 60 kann eine schmale, langgestreckte Form mit zwei Enden 64 und zwei Endbereichen 63 aufweisen. Die beiden Endbereiche 63 können bis zu 50% der Gesamtlänge L des gesamten Kopfbandes 60 ausmachen. In bevorzugten Ausführungsformen machen die beiden Endbereiche 63 zusammen ca. 25% der Gesamtlänge L des gesamten Kopfbandes 60 aus. An einem und/oder an beiden Endbereichen 63 des Kopfbandes 60 können Befestigungselemente 62 angeordnet sein.

In manchen Ausführungsformen können beide Endbereiche 63 des Kopfbandes 60 ein oder mehrere Befestigungselemente 62 umfassen. Beispielsweise können die Endbereiche 63 des Kopfbandes 60 einen oder mehrere Klettverschlüsse und/oder Haken und Ösen und/oder Knöpfe und Knopflöcher und/oder Druckknöpfe und/oder Schnallen oder ähnliche Elemente umfassen. Mit diesen Befestigungselementen 62 kann das Kopfband 60 an dem einen oder mehreren Bügeln 20 reversibel befestigt werden. Die Befestigungselemente 62 sind derart eingerichtet und ausgebildet, dass eine Längenverstellung des Kopfbandes möglich ist.

Die Endbereiche 63 des Kopfbandes 60 weisen bevorzugt einen oder mehrere Klettverschlüsse auf. Zu diesem Zweck kann an einem oder an beiden Endbereichen 63 ein Klettbereich mit flexiblen Widerhaken angeordnet sein, die mit dem Grundmaterial des Kopfbandes 60 eine belastungsfähige, feste und reversible Verbindung eingehen können (nicht dargestellt).

Zur Befestigung des Kopfbandes 60 an die Bügel können die beiden Enden 64 und die Endbereiche 63 des Kopfbandes 60 durch die Durchbrüche 26 der Bügel 20 hindurchführen. Sodann kann das Kopfband 60 schlaufenförmig gebogen werden, so dass die Endbereiche 63 mit anderen Bereichen des Kopfbandes 60 verbunden werden können (siehe beispielsweise Fig. 7). Bevorzugt kann die Verbindung über einen Klettverschluss gehalten werden.

Es ist auch denkbar, dass mindestens eines der beiden Enden 64 und/oder Endbereiche 63 des Kopfbandes 60 zum Schutz vor Verlust irreversibel mit einem der Bügel 20 verbunden ist. Bevorzugt ist mindestens einer der beiden Endbereiche 63 des Kopfbandes 60 wie oben beschrieben längenverstellbar am Bügel 20 befestigt.

**Fig. 10** zeigt einen Teilbereich des Bügels 20 in verschiedenen Ausführungsformen. Aus Fig. 10 wird ersichtlich, dass der Endabschnitt 37 des Bügels 20 zum Ende 38 hin unterschiedlich ausgebildet sein kann. In manchen Ausführungsformen kann der Endabschnitt 37 eine konstante Breite B aufweisen (Fig. 10A). In alternativen Ausführungsformen kann zumindest ein Teilbereich des Endabschnittes 37 eine besondere Ausgestaltung aufweisen. Beispielsweise kann der Endabschnitt 37 zumindest abschnittsweise gegenüber der grundsätzlich konstanten Breite B verbreitert ausgebildet sein. Ein verbreitert ausgebildeter Endabschnitt 37 kann vorteilhaft für die Befestigung des Hinterohrelements 40 am Bügel 20 sein.

Der Endabschnitt 37 kann beispielsweise zumindest abschnittsweise zu einem Ankerelement 25 verbreitert ausgebildet sein. Das Ankerelement 25 kann derart ausgebildet sein, dass das Ende 38 pfeilförmig verbreitert ist (Fig. 10B). Die pfeilartige Verbreiterung kann ein ungewolltes Abrutschen des Hinterohrelements 40 vom Bügel 20 effektiv verhindern. Das Ankerelement 25 ist nicht auf die pfeilartige Form beschränkt. Beispielsweise kann das Ankerelement 25 auch kreisförmig und/oder oval und/oder oder viereckig ausgebildet sein. Das Ankerelement 25 kann auch jegliche andere geeignete Geometrie aufweisen, die die Kopplung des Hinterohrelements 40 an den Bügel 30 erlaubt und gleichzeitig das ungewollte Abrutschen verhindert.

Alternativ oder zusätzlich kann der Endabschnitt 37 des Bügels 20 ein oder mehrere Fixierungselemente 24 umfassen, die die Befestigung des Hinterohrelements 40 am Bügel 20 positiv beeinflussen können (Fig. 10C). Die Fixierungselemente 24 am Bügel 20 können in Form von Knöpfen, Pilzknöpfen, Haken, Stäben, Nieten, Klettband mit Widerhaken und/oder Schlaufen oder ähnliches ausgebildet sein. Entsprechende ein oder mehrere Gegenpart-Elemente, also Knopflöcher, Löcher, Ösen, Schlaufen, Klettband mit Widerhaken und/oder Schlaufen oder ähnliches können sich auf und/oder an den Enden 44 oder Endbereichen 43 des Hinterohrelements 40 befinden (nicht gezeigt).

Die Fixierungselemente 24 können auch durch ein oder mehrere Verdickungen und/oder Einkerbungen oder ähnlichen Strukturen auf den Bügeln 20 ausgebildet sein, die eine Rastung auf dem Bügel 20 zulassen (nicht gezeigt).

Die Befestigung eines oder mehrerer Fixierungselemente 24 mit einem entsprechenden Gegenpart-Element kann die Befestigung des Hinterohrelements 40 an den Bügeln 20 verstärken und/oder sichern. Über die Anordnung mehrerer Fixierungselemente 24 am Bügel, die sich an unterschiedlichen Positionen des Bügels 20 befinden, kann eine weitere Längenverstellung des Verbindungselements 10 ermöglicht sein.

Die Anordnung zusätzlicher Fixierungselemente 24 am Endabschnitt 37 des Bügels 20 kann insbesondere bei einer Überdruckbeatmung von Vorteil sein, da in diesem Falle eine starke Kraft auf das Verbindungselement 10 wirkt. Zusätzliche Fixierungselemente 24 kann die Befestigung des Hinterohrelements 40 an den Bügeln 20 sichern und stabilisieren.

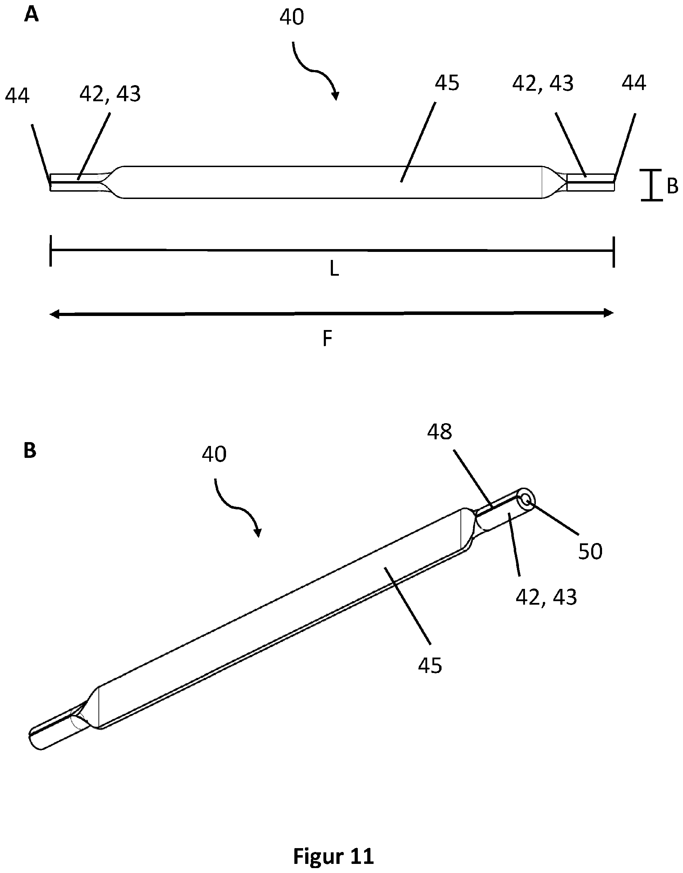

**Fig. 11 bis 14** zeigen unterschiedliche Ausführungsformen für das Hinterohrelement 40 des Verbindungselements 10 aus Fig. 3.

In der Ausführungsform nach Fig. 3 ff ist ein einziges Hinterohrelement 40 vorgesehen. Das eine Hinterohrelement 40 kann aus einem oder mehreren Fertigungsteilen gefertigt sein. Das Hinterohrelement 40 umfasst in dieser konkreten Ausführungsform mindestens zwei Endbereiche 43. Zwischen den beiden Endbereichen 43 kann mindestens ein Mittelstück 45 ausgebildet sein.

Die beiden Endbereiche 43 können bis zu 100 % der Gesamtlänge L des gesamten Hinterohrelementes 40 ausmachen, so dass das Hinterohrelement 40 ohne Mittelstück 45 ausgebildet ist (nicht gezeigt). In alternativen Ausführungsformen ist zwischen den beiden Endbereichen 43 ein Mittelstück 45 ausgebildet, dass unterschiedlich lang ausgebildet sein kann. Beispielsweise können die beiden Endbereiche 43 zusammen bis zu 50 % der Gesamtlänge L des gesamten Hinterohrelementes 40 ausmachen und das Mittelstück 45 die übrigen 50 %. Das Mittelstück 45 kann auch kürzer oder länger ausgebildet sein.

In bevorzugten Ausführungsformen machen die Endbereiche 43 höchstens 25% oder weniger der Gesamtlänge L des gesamten Hinterohrelementes 40 aus. Das Hinterohrelement 40 kann über seine beiden Endbereiche 43 an die Endabschnitte 37 der beiden Bügel 20 gekoppelt werden.

Das Mittelstück 45 kann bevorzugt mindestens 50 % der Gesamtlänge L des gesamten Hinterohrelementes 40 ausmachen. In bevorzugten Ausführungsformen macht das Mittelstück mindestens 75% oder mehr der Gesamtlänge L des gesamten Hinterohrelementes 40 aus.

Die Länge L des Hinterohrelementes 40 ist in den Ausführungsbeispielen nach Fig. 11 bis 14 wesentlich größer als die Breite B des Hinterohrelementes 40. Beispielsweise beträgt das Verhältnis der Länge L zur Breite B des Hinterohrelementes 40 mindestens 5:1. In manchen Ausführungsformen beträgt das Verhältnis der Länge L zur Breite B des Hinterohrelementes 40 mindestens 20:1. In einer bevorzugten Ausführungsform beträgt das Verhältnis der Länge L zur Breite B des Hinterohrelementes 40 30:1 oder mehr. Das Hinterohrelement 40 weist in den Ausführungsbeispielen gemäß der Figuren 11 bis 14 bevorzugt eine langgestreckte, schmale Form auf.

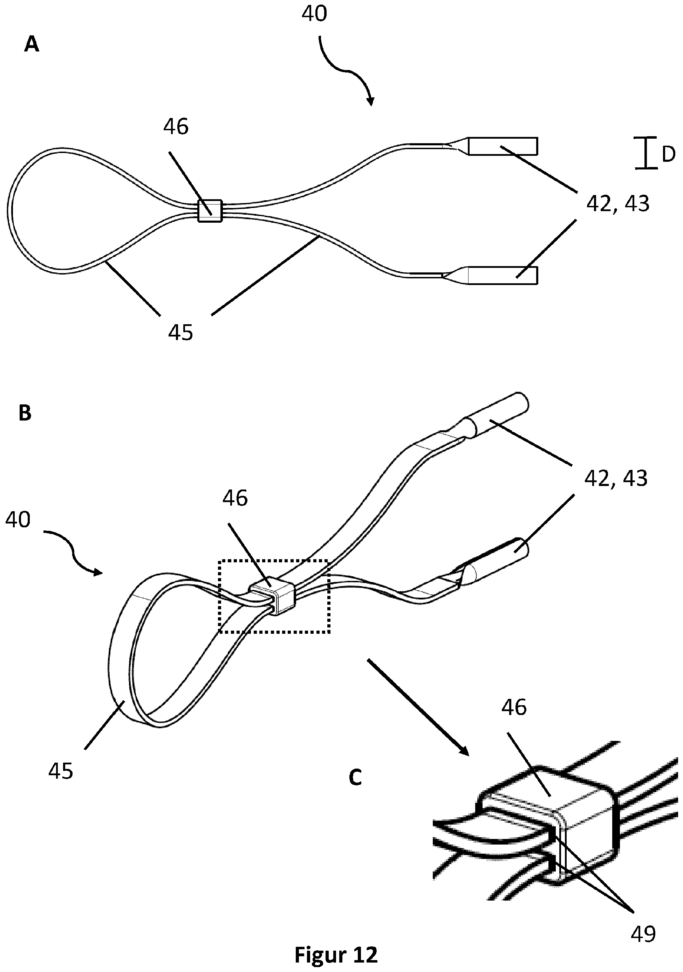

Die Breite B des Hinterohrelementes 40 kann konstant oder variierend ausgebildet sein. In einer beispielhaften Ausführungsform kann die Breite B des Hinterohrelementes 40 im Wesentlichen konstant ausgebildet sein (Fig. 11, 13, 14). In manchen Ausführungsformen kann die Breite B des Hinterohrelementes 40 auch variierend ausgebildet sein (Fig. 12). Die Breite B des Hinterohrelementes 40 kann in einem Bereich zwischen 0,2 mm bis 30 mm liegen. Bevorzugt liegt die Breite B des Hinterohrelementes 40 zwischen 0,5 mm und 25 mm, besonders bevorzugt zwischen 7 und 25 mm.

Die Länge L des Hinterohrelementes 40 kann in einem Bereich zwischen 50 bis 400 mm liegen. Bevorzugt liegt die Länge L des Hinterohrelementes 40 zwischen 100 und 300 mm. In einem konkreten Ausführungsbeispiel beträgt die Länge L des Hinterohrelementes 40 beispielhaft 210 mm.

Bevorzugt ist das Hinterohrelement 40 aus einem elastischen Material gefertigt. Das Hinterohrelement 40 kann aus zumindest einem Material ausgewählt aus der Gruppe Silikone, Thermoplastischen Elastomere (TPE), Neoprene oder Textilien gefertigt sein. Denkbare Textilien für das Hinterohrelement können Baumwolle, Seide und/oder synthetischen Fasern wie beispielsweise Polyester, Polyurethan, Neopren, Spandex, Nylon sein. Bevorzugt ist das Hinterohrelement 40 aus Silikon gefertigt.

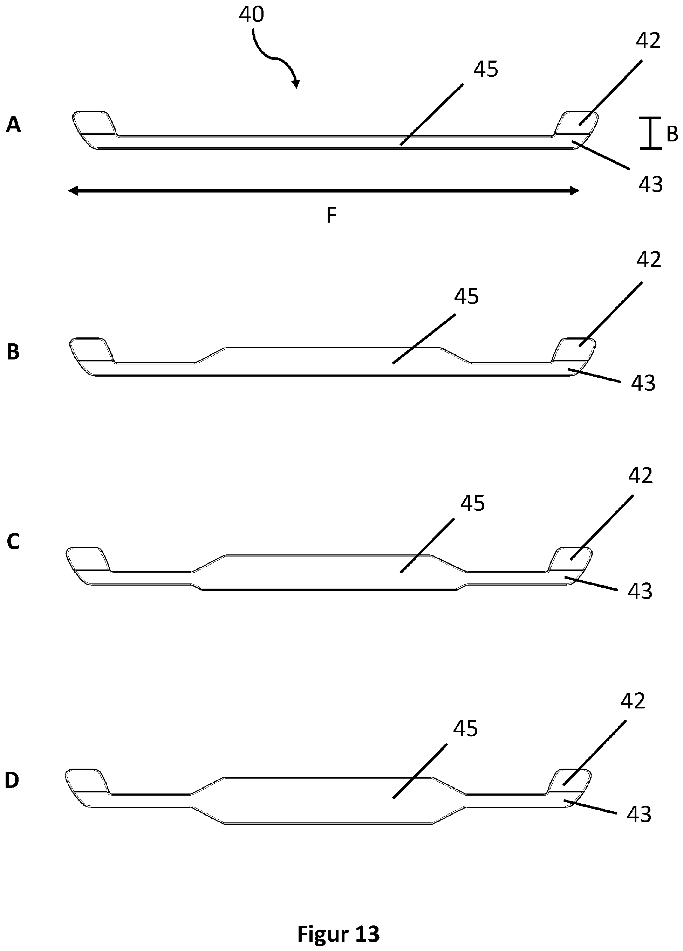

Das Hinterohrelement 40 kann an die Bügel 20 gekoppelt werden. Das Hinterohrelement 40 kann mindestens ein, bevorzugt zwei Halteelemente 42 umfassen. Die Halteelemente 42 können innerhalb der Endbereiche 43 ausgebildet und/oder durch die Endbereiche 43 ausgebildet sein (siehe Fig. 11, 12, 14). In alternativen Ausführungsformen können die Halteelemente 42 auch an den Endbereichen 43 angeformt sein (siehe Fig. 13). Über die Halteelemente 42 kann das Hinterohrelement 40 an die Bügel 20 gekoppelt werden. Die Halteelemente 42 können bevorzugt an die Endabschnitten 37 der Bügel 20 gekoppelt werden.

Die Kopplung des Hinterohrelements 40 an die Bügel 20 kann irreversibel und/oder reversibel sein. In manchen Ausführungsformen ist denkbar, dass ein Halteelement 42 irreversibel an den einen Bügel 20 gekoppelt ist und das andere Halteelement 42 reversibel an den zweiten Bügel 20 (nicht gezeigt). Es ist auch denkbar, dass die Hinterohrelemente 40 integrative Bestandteile der Bügel 20 sind und/oder dass Bügel 20 und Hinterohrelemente 40 einstückig gefertigt sind.

Die Kopplung des Hinterohrelements 40 an die Bügel 20 ist bevorzugt reversibel ausgebildet. Die Kopplung des Hinterohrelements 40 an die Bügel 20 kann beweglich und/oder unbeweglich ausgebildet sein. Bevorzugt ist die Kopplung des Hinterohrelements 40 an die Bügel 20 beweglich ausgebildet. Durch eine bewegliche Kopplung des Hinterohrelements 40 an die Bügel 20 kann die Positionierung des Hinterohrelements 40 innerhalb des Verbindungselements 10 verändert werden (siehe Fig. 8A und Fig. 8B).

**Fig. 11** zeigt ein einfaches Ausführungsbeispiel für das Hinterohrelement 40 des Verbindungselements 10 aus Fig. 3, wobei Fig. 11 A eine Ansicht von der Seite und Fig. 11 B eine perspektivische Ansicht zeigt.

In dem konkreten Ausführungsbeispiel nach Fig. 11 kann die Länge L des Mittelstücks 45 bis zu 90% und die Länge L der beiden Endbereiche 43 zusammen ca. 10% der Gesamtlänge L des Hinterohrelementes 40 ausmachen. In dem Ausführungsbeispiel nach Fig. 11 kann die Breite B des Hinterohrelements 40 im Wesentlichen konstant sein. Die Breite B des Mittelstücks 45 kann konstant ausgebildet sein.

Die Endbereiche 43 können jeweils zumindest abschnittsweise als Halteelemente 42 in Form von länglichen Hohlkörpern ausgebildet sein. Die länglichen Hohlkörper können beispielsweise einen runden und/oder ovalen oder eckigen Querschnitt aufweisen. Die länglichen Hohlkörper können ausgebildet werden, indem die Endbereiche 43 jeweils zumindest abschnittsweise der Länge L nach zu einem geschlossenen Profil verbunden werden. Die Verbindung der Endbereiche 43 zu einem Hohlkörper kann über nähen, schweißen, kleben oder ähnliches erfolgen, so dass der Endbereich 43 eine Verbindungsnut 48 aufweisen kann. Die Verbindung des Endbereichs 43 zu einem Hohlkörper ist in der Regel und bevorzugt irreversibel ausgebildet.

Die derart ausgebildeten Halteelemente 42 können auf die Endabschnitte 37 der Bügel 20 aufgesteckt werden (nicht gezeigt). Die Endabschnitte 37 der Bügel 20 befinden sich sodann zumindest abschnittsweise im Lumen 50 der Halteelemente 42. Dort halten die Halteelemente 42 das Hinterohrelement 40 unter Vorspannung durch Reibung an den Endabschnitten 37 der Bügel 20.

Damit das Verbindungselement 10 gut am Kopf 95 eines Anwenders hält, muss das Verbindungselement 10 um einen gewissen Grad kleiner eingestellt sein als der Umfang des Kopfes 95 groß ist. Dadurch kann in einem Anwendungszustand auf das Hinterohrband 40 eine **Zugkraft F** einwirken. Die Zugkraft F verläuft in der Regel in Richtung der Länge L des Hinterohrbandes 40. Im Ausführungsbeispiel gemäß Fig. 11 sind die Halteelemente 42 entlang der Zugkraft F in einer Ebene mit dem Mittelstück 45.

Die Halterung kann alternativ oder zusätzlich durch Fixierungselemente 24 und/oder Ankerelemente 25 an den Endabschnitten 37 der Bügel ausgebildet und/oder unterstützt werden (siehe Fig. 10). Zu diesem Zweck kann mindestens ein Endbereich 43 des Hinterohrelements 40 mindestens ein Knopfloch oder Loch oder eine Öse oder eine Schlaufe oder ein Klettband mit Widerhaken und/oder Schlaufen oder ähnliches aufweisen. In manchen Ausführungsformen ist es auch denkbar, dass der Endbereich 43 mehrere Knopflöcher oder Löcher oder Ösen oder Schlaufen oder ähnliches aufweist, so dass eine Längenverstellung möglich ist (nicht gezeigt).

Eine zusätzliche Fixierung bietet den Vorteil, dass das Hinterohrelement 40 fest mit dem Bügel 20 verbunden und ein ungewolltes Verrutschen auf dem Bügel 20 verhindert werden kann. Dies ist insbesondere bei der Verwendung von Atemmasken 90 für die Überdruckbeatmung von Vorteil, da so die Zugkraft F zum Abdichten der Atemmaske 20 über das Hinterohrelement 40 verstärkt werden kann.

**Fig. 12** zeigt ein weiteres Ausführungsbeispiel für das Hinterohrelement 40 des Verbindungselements 10 aus Fig. 3. Das in Fig. 12 dargestellte Hinterohrelement 40 entspricht im Wesentlichen dem in Fig. 11 dargestellten Hinterohrelement 40, zeigt jedoch, dass das Hinterohrelement 40 in manchen Ausführungsformen wesentlich verlängert ausgebildet sein kann. Das Hinterohrelement 40 kann um bis zu 200% länger ausgebildet sein als zuvor beschrieben.

Das Hinterohrelement 40 kann in dieser Ausführungsform zudem mindestens eine Öse 46 umfassen. Die Öse 46 kann ein oder mehrere Öffnungen 49 aufweisen (siehe Detailansicht der Fig. 12C). Durch die ein oder mehreren Öffnungen 49 kann das Hinterohrelement 40 derart verlaufen, dass das Mittelstück 45 zumindest abschnittsweise zu einer Schlaufe geformt ist. Durch Verschieben der Öse 46 kann die Länge L des Hinterohrelements 40 eingestellt werden. Das Hinterohrelement 40 ist somit in sich längenverstellbar. Die Öse 46 hält das Hinterohrelement 40 durch Reibung und/oder unter Vorspannung.

Die Öse 46 kann alternativ oder zusätzlich einen Federmechanismus und/oder einen Rastmechanismus beinhalten (nicht gezeigt). Der Federmechanismus der Öse 46 kann eine Feder und ein Betätigungselement umfassen. Durch den Federmechanismus kann das Hinterohrelement 40 klemmend in seiner Position fixiert werden. Die fixierte Position kann durch Druck auf das Betätigungselement aufgehoben werden, um ein Verstellen der Position der Öse 46 und somit eine Längenverstellung zu ermöglichen. Das in Fig. 12 gezeigte Ausführungsbeispiel bietet den Vorteil einer sehr flexiblen, einfachen, schnellen und sicheren Längenverstellung des Hinterohrelements 40.

**Fig. 13** zeigt ein weiteres Ausführungsbeispiel für das Hinterohrelement 40 des Verbindungselements 10 aus Fig. 3. In Fig. 13 sind beispielhafte Ausführungsformen für das Hinterohrelement 40 dargestellt, bei denen die Breite B des Mittelstücks 45 gegenüber der Breite B der Endbereiche 43 breiter und/oder schmaler ausgebildet sein kann. Das Mittelstück 45 kann in diesen Ausführungsbeispielen ca. 50% der Gesamtlänge L des Hinterohrelementes 40 ausmachen. Die Endbereiche 43 können in diesen Ausführungsbeispielen zusammen ca. 50% der Gesamtlänge L des Hinterohrelementes 40 ausmachen. Das Mittelstück 45 kann auch kürzer ausgebildet sein als dargestellt.

Fig. 13 A zeigt eine einfache Ausführungsform eines Hinterohrelementes 40 mit einer im Wesentlichen konstanten Breite B. Lediglich die Halteelemente 42 sind breiter ausgebildet als die grundsätzliche Breite B des Hinterohrelementes 40. Fig. 13 B bis D zeigen, dass das Mittelstück 45 gegenüber der Breite B der Endbereiche 43 breiter ausgebildet sein kann. Ein breit ausgebildetes Mittelstück 45 kann sich positiv auf die Bequemlichkeit und die Stabilität des Hinterohrelements 40 auswirken.

Aus Fig. 13 wird weiterhin ersichtlich, dass die Halteelemente 42 an den Endbereichen 43 angeformt sein können. Die Halteelemente 42 können oberhalb und/oder unterhalb der Endbereiche 43 angeformt sein. Die Halteelemente 42 können auch derart ausgebildet sein, dass sie in einer Ebene mit den Endbereichen 43 angeordnet sind (siehe Fig. 11, 12, 14).

In den Ausführungsformen gemäß Fig. 13 sind die Halteelemente 43 derart an den Endbereichen 43 angeformt, dass die Halteelemente 42 entlang der Zugkraft F nicht in einer Ebene mit dem Mittelstück 45 liegen. Der Kippmoment im Halteelement 42 bewirkt ein stärkeres Klemmen und damit für einen besseren Halt des Hinterohrelements 40 am Bügel 20.

Ein weiterer Vorteil dieser Ausführungsformen liegt darin, dass die Lage des Hinterohrelements 40 im Anwendungszustand variiert werden kann. Das Hinterohrelement 40 kann so gedreht werden, dass die es in einem Anwendungszustand im (nicht gezeigten) Nacken verläuft (siehe Fig. 8A) oder über den (nicht gezeigten) Hinterkopf verläuft (siehe Fig. 8B).

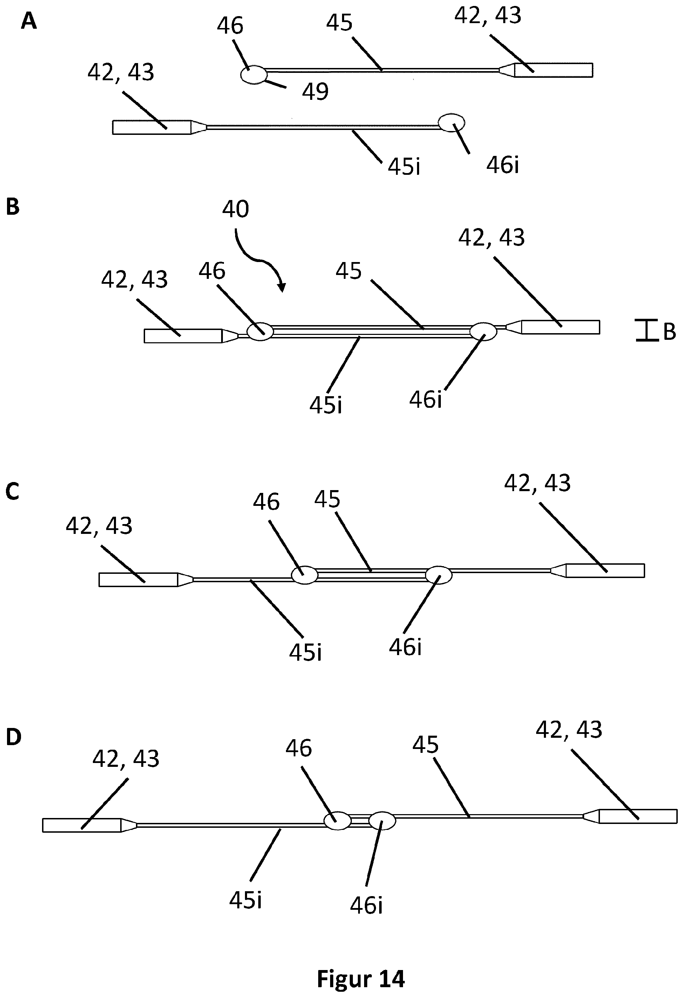

**Fig. 14** zeigt ein alternatives Ausführungsbeispiel für das Hinterohrelement 40 des Verbindungselements 10 aus Fig. 3.

Das Hinterohrelement 40 gemäß Fig. 14 kann aus mehreren, beispielsweise zwei Fertigungsteilen gefertigt sein, die zur Herstellung eines fertigen Bauteils zu einem Hinterohrelement 40 zusammengefügt werden können. Durch die zweigeteilte Fertigung kann ein Mechanismus zur Längenverstellung ausgebildet sein. Die Darstellung in Fig. 14 A zeigt das Hinterohrelement 40 in einem teilweise auseinandergebauten Zustand. Fig. 14 B-D zeigt das Hinterohrelement 40 in einem zusammengebauten, anwendungsfertigen Zustand.

Fig. 14A zeigt, dass das Hinterohrelement 40 aus zwei baugleichen Fertigungsteilen gefertigt sein kann. Jedes der beiden Fertigungsteile enthält einen Endbereich 43 mit einem Halteelement 42, ein Mittelstück 45 und eine Öse 46. Die Öse 46 kann jeweils fest mit dem einen Ende des Mittelstückes 45 verbunden sein. Der Endbereich 42 kann fest mit dem anderen Ende des Mittelstücks 45 verbunden sein. Die Öse 46 kann mindestens eine Öffnung 49 aufweisen. Die Öffnung 49 kann einseitig in der Öse 46 angeordnet sein. Die Öffnung 49 kann beidseitig in der Öse 46 angeordnet sein und einen Durchgangskanal durch die Öse 46 ausbilden.

Das in Fig. 14 B-D dargestellte zusammengebaute Hinterohrelement 40 zeigt, dass das Hinterohrelement 40 in manchen Ausführungsformen einen Längenverstell-Mechanismus umfassen kann. Zur Einrichtung des Längenverstell-Mechanismus kann das Hinterohrelement 40 mindestens zwei oder mehr Ösen 46, 46i und/oder zwei oder mehr Mittelstücke 45, 45i umfassen.

Für das konkrete Ausführungsbeispiel gemäß Fig. 14 weist das Hinterohrelement 40 bevorzugt zwei Ösen 46, 46i und zwei Mittelstücke 45, 45i auf. Die erste Öse 46 kann mit dem ersten Mittelstück 45 fest verbunden sein. Das zweite Mittelstück 45i kann die erste Öse 46 beweglich durch die Öffnung 49 durchlaufen. Die zweite Öse 46i kann mit dem zweiten Mittelstück 45i fest verbunden sein. Das erste Mittelstück 45 kann die zweite Öse 46i beweglich durch die Öffnung 49 durchlaufen. Durch eine derartige zweigeteilte Ausführungsform kann eine einfache und sichere Längenverstellung des Hinterohrelements 40 ermöglicht sein. Somit ist das Hinterohrelement 40 in sich längenverstellbar.

Die Ösen 46, 46i halten das jeweils durch die Öffnung 49 der Öse 46, 46i durchlaufende Mittelstück 45, 45i durch Reibung und/oder unter Vorspannung und/oder über einen Federmechanismus. Es ist auch denkbar, dass die Mittelstücke 45, 45i Verdickungen aufweisen, die eine gerastete Längenverstellung ermöglichen (nicht gezeigt).

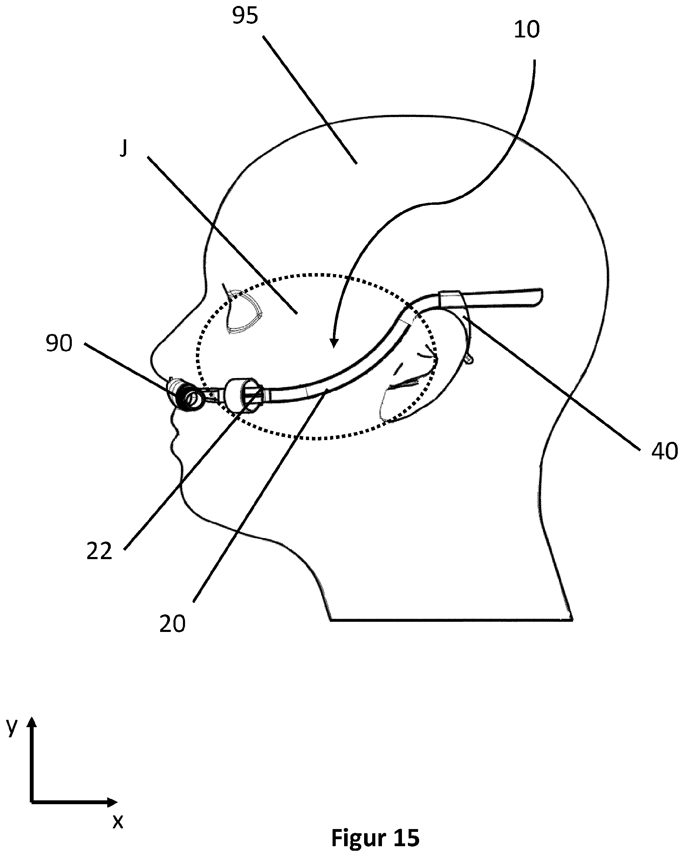

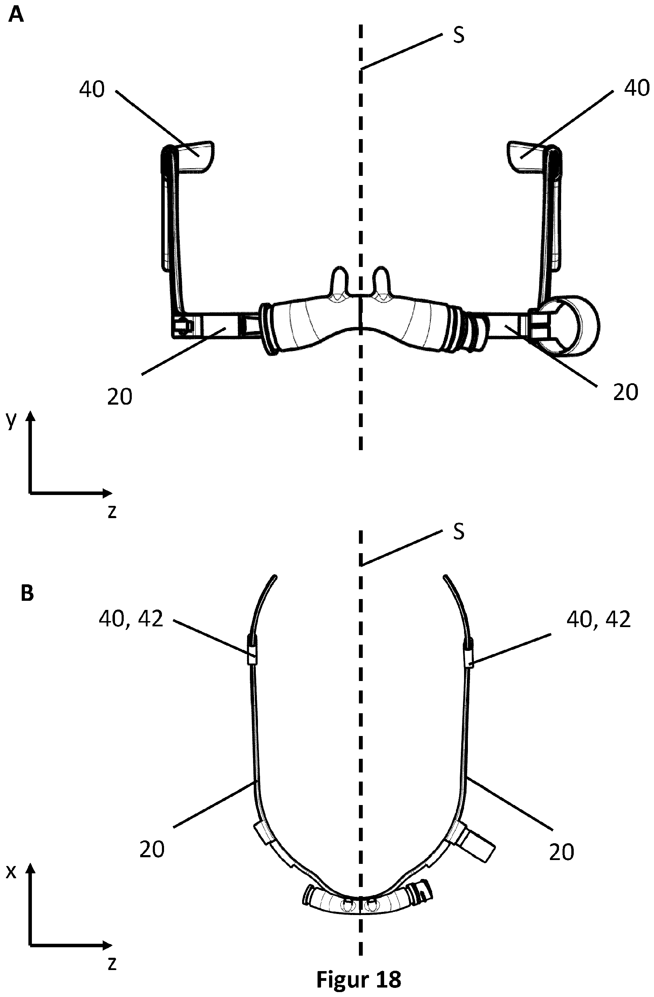

**Fig. 15** zeigt eine schematische Seitenansicht einer alternativen Ausführungsform des erfindungsgemäßen Verbindungselements 10 in einem Anwendungszustand am Kopf 95 eines Anwenders. **Fig. 16 bis 18** zeigen unterschiedliche Ansichten des erfindungsgemäßen Verbindungselements 10 aus Fig. 15, wobei **Fig. 16** eine Ansicht des Verbindungselements 10 von der Seite, **Fig. 17** eine perspektivische Ansicht des Verbindungselements 10, **Fig. 18A** eine Ansicht des Verbindungselements 10 von vorne und Fig. **18B** eine Ansicht des Verbindungselements 10 von oben zeigen.

In der alternativen Ausführungsform umfasst das Verbindungselement 10 mindestens einen Bügel 20 und mindestens ein Hinterohrelement 40. Bevorzugt umfasst das Verbindungselement 10 zwei Bügel 20 und zwei Hinterohrelemente 40.

Das Verbindungselement 10 der alternativen Ausführungsform gemäß Fig. 15 ff und das Verbindungselement 10 gemäß Fig. 3 ff umfassen beide die erfindungsgemäßen, zuvor ausführlich beschriebenen Bügel 20. Die Bügel 20 umfassen das Koppelsystem 22 zur Kopplung an das Patienteninterface 90 und den Endabschnitt 37 zur Kopplung des mindestens einen Hinterohrelements 40. Die Kopplung des Hinterohrelements 40 an die Bügel 20 erfolgt bevorzugt über die Halteelemente 42. Die Halteelemente 42 können ausgebildet und eingerichtet, eine Längenverstellung zuzulassen und/oder eine Fixierung und/oder Verankerung am Bügel 20 zu ermöglichen. Ein Kopfband 60 ist in der alternativen Ausführungsform denkbar aber nicht notwendig.

Aus Fig. 15 wird ersichtlich, dass das Verbindungselement 10 der alternativen Ausführungsform in einem Anwendungszustand ähnlich zum Verbindungselements 10 gemäß Fig. 3 ff derart an dem Kopf 95 eines Patienten angeordnet ist, dass der Bügel 20 zumindest abschnittsweise zwischen der Nase und dem Ohr angeordnet ist. Hierbei befindet sich der Bügel 20 zumindest abschnittsweise in der Jochbeinregion J. Der Bügel 20 ist entsprechend der oben ausführlich dargestellten Ausführungsform ausgebildet.

Die alternative Ausführungsform gemäß Fig. 15 ff unterscheidet sich zu den Ausführungsformen gemäß Fig. 3 ff in der Ausgestaltung des Hinterohrelements 40. In der Ausführungsform nach Fig. 15 ff sind mindestens zwei Hinterohrelemente 40 vorgesehen, die jeweils an einen der mindestens zwei Bügel 20 gekoppelt werden können.

Das Patienteninterface 90 kann als Highflow-Interface, kurz HF-Interface ausgebildet sein. Über das HF-Interface 90 kann der Patient eine Highflow-Sauerstofftherapie, kurz HFOT, erhalten. Die HFOT stellt eine nicht-invasive Form der Atemunterstützung und/oder Atemtherapie dar. Bei einer HFOT kann dem Patienten Sauerstoff und/oder ein sauerstoffhaltiges Gasgemisch zugeführt werden. Der Sauerstoff und/oder das Gasgemisch kann mit einem sehr hohen Gasfluss von bis zu 60 l/min appliziert werden. Das HF-Interface ist üblicherweise als Nasenkanüle und/oder Nasenbrille ausgebildet und kann Nasalprongs 92 umfassen. Beispielsweise ist das HF-Interface als Nasenbrille mit mindestens einem, bevorzugt zwei Nasalprongs 92 ausgebildet. Die Nasalprongs 92 (siehe Fig.16) können zumindest teilweise in die Nase eingeführt werden (nicht gezeigt).

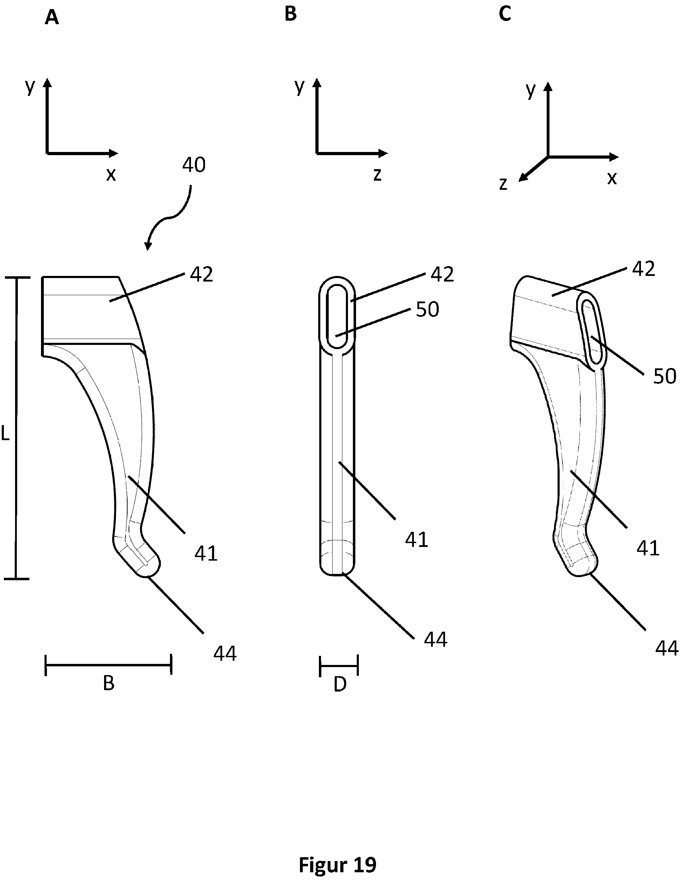

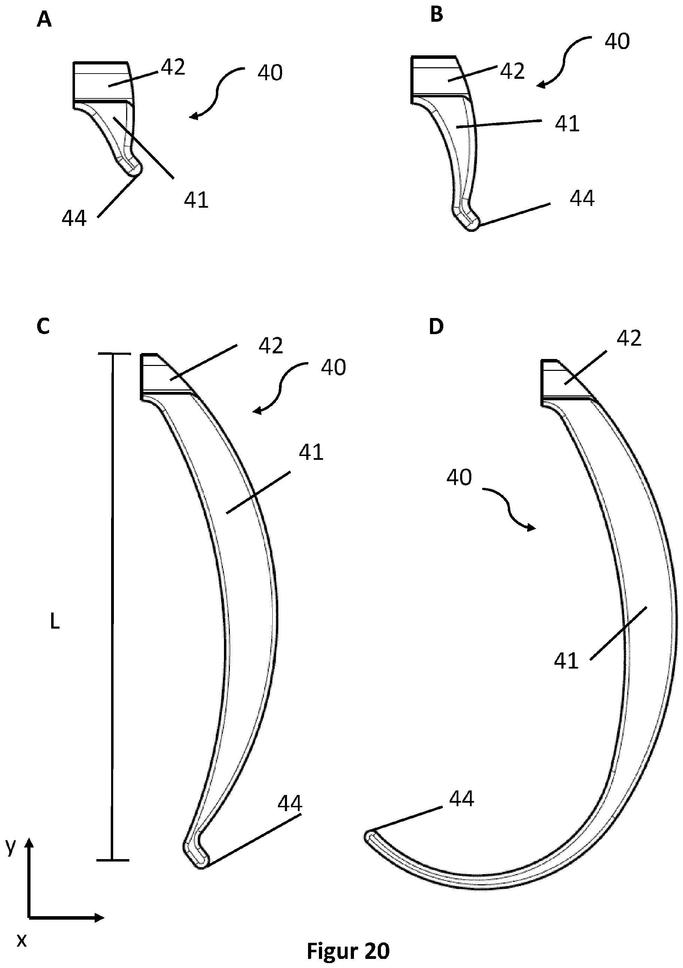

Die **Figuren 19** **und** **20** zeigen Ausführungsbeispiele für das Hinterohrelement 40 des erfindungsgemäßen Verbindungselements 10 gemäß Fig. 15 ff. Entgegen den Ausführungsbeispielen gemäß Fig. 11 bis 14 kann das Hinterohrelement 40 kürzer ausgebildet sein. Das Verhältnis der Länge L zur Breite B der Hinterohrelemente 40 kann in diesen Ausführungsbeispielen jeweils weniger als 20:1, bevorzugt weniger als 10:1, besonders bevorzugt weniger als 5:1 betragen.

**Fig. 19** zeigt ein einfaches Ausführungsbeispiel für das Hinterohrelement 40 des erfindungsgemäßen Verbindungselements 10 gemäß Fig. 15, wobei Fig. 19 A eine Ansicht von der Seite, Fig. 19 B eine Ansicht von vorne und Fig. 19 C eine perspektivische Ansicht zeigt.

In der Ausführungsform nach Fig. 15 ff ist jeweils ein Hinterohrelement 40 je Bügel 20 vorgesehen. Bevorzugt weist das Verbindungselement 10 zwei Bügel 20 auf mit jeweils einem Hinterohrelement 40. Fig. 19 zeigt ein Hinterohrelement 40, das in einem Anwendungszustand hinter dem Ohr angeordnet ist (siehe Fig. 15). Aus Fig. 19 wird ersichtlich, dass die Länge L des Hinterohrelementes 40 etwas größer als die Breite B sein kann. Beispielsweise beträgt das Verhältnis der Länge L zur Breite B des Hinterohrelementes 40 weniger als 20:1, bevorzugt weniger als 10:1, besonders bevorzugt weniger als 5:1. Das Verhältnis der Länge L zur Breite B des Hinterohrelementes 40 beträgt in dieser konkreten Ausführungsform beispielhaft etwa 3:1.

Das Hinterohrelement 40 umfasst in dieser konkreten Ausführungsform ein Halteelement 42. Das Halteelement 42 ist als Hohlkörper ausgebildet und umfasst mindestens ein Lumen 50. Die Hohlkörper und das Lumen 50 können beispielsweise einen runden und/oder ovalen oder eckigen Querschnitt aufweisen. Das Hinterohrelement 40 kann in manchen Ausführungsformen nur aus dem Halteelement 42 bestehen (nicht gezeigt). In alternativen Ausführungsformen kann das Hinterohrelement 40 zudem ein Endstück 41 umfassen (Fig. 19/20).

Zur Herstellung eines Anwendungszustandes kann das Hinterohrelement 40 an den Bügel 20 gekoppelt werden. Die Kopplung erfolgt über das Halteelement 42, das an den Endabschnitt 37 eines Bügels 20 gekoppelt werden kann. Bevorzugt wird an beide Bügel 20 jeweils ein Hinterohrelement 40 gekoppelt. Der Endabschnitt 37 eines Bügels 20 befindet sich sodann zumindest abschnittsweise im Lumen 50 eines Halteelements 42. Dort halten die Halteelemente 42 das Hinterohrelement 40 unter Vorspannung durch Reibung an den Endabschnitten 37 der Bügel 20. Die Halterung kann alternativ oder zusätzlich - wie weiter oben hierin zur Fig. 10 beschrieben - durch Fixierungselemente 24 und/oder Ankerelemente 25 an den Endabschnitten 37 der Bügel und mit entsprechenden Gegenpart-Elementen an den Hinterohrelementen 40 ausgebildet und/oder unterstützt werden.

Die Kopplung der Hinterohrelemente 40 an die Bügel 20 kann eine Anpassung des Verbindungselements 10 an die jeweilige Kopfform zulassen. Die Positionierung der Hinterohrelemente 40 an den Endabschnitten 37 der Bügel 20 kann bevorzugt variabel einstellbar sein. Für kleinere Kopfformen kann das Hinterohrelement 40 auf dem Bügel 20 in Richtung des Anfangs 30 verschoben werden. Für größere Kopfformen kann das Hinterohrelement 40 auf dem Bügel 20 in Richtung des Endes 38 verschoben werden. Dies bietet den Vorteil einer sehr einfachen und schnellen Größenanpassung des Verbindungselements 10.

Im Gegensatz zum Hinterohrelement 40 gemäß Fig. 3 ff verläuft das Hinterohrelement 40 gemäß Fig. 15 ff. in einem Anwendungszustand nicht über den Hinterkopf eines Anwenders. Vielmehr ist das Hinterohrelement 40 gemäß Fig. 15 ff in einem Anwendungszustand am und/oder hinter dem Ohr angeordnet. Das Hinterohrelement 40 kann wesentlich kürzer ausgebildet sein als in den Ausführungsformen gemäß Fig. 11 bis 14. Die Länge L des Hinterohrelements 40 kann durch unterschiedlich lang ausgebildete Mittelstücke 45 und/oder Endstücke 41 variiert werden.

Das Endstück 41 gemäß Fig. 19/20 kann wesentlich kürzer ausgebildet sein als das entsprechende Mittelstück 45 der Ausführungsformen gemäß Fig. 11-14. Fig. 19 zeigt ein Hinterohrelement 40, das in einem Anwendungszustand hinter dem Ohr angeordnet ist (siehe Fig. 15). Die Länge L des Endstücks 41 kann in einem Bereich zwischen 10 mm bis 150 mm liegen. Bevorzugt liegt die Länge L des Endstücks 41 zwischen 15 und 100 mm. In dem konkreten Ausführungsbeispiel gemäß Fig. 19 beträgt die Länge L des Endstücks 41 beispielhaft 32 mm.

**Fig. 20** zeigt alternative Ausführungsbeispiele für das Hinterohrelement 40 des erfindungsgemäßen Verbindungselements 10 gemäß Fig. 15 von der Seite. Aus Fig. 20 wird ersichtlich, dass das Endstück 41 des Hinterohrelements 40 unterschiedliche Längen L aufweisen kann. Fig. 20 A zeigt ein besonders kurzes Ausführungsbeispiel eines Hinterohrelements 40 mit einer Gesamtlänge L von 20 mm.

Das Hinterohrelement 40 kann unterschiedliche Längen L aufweisen (Fig. 20 A-D). Beispielsweise kann das Hinterohrelement 40 derart lang und gebogen ausgebildet sein, dass es das Ohr in einem Anwendungszustand (nicht gezeigt) derart umschließt, dass das Verbindungselement 10 einen besonders vorteilhaften Halt erfährt (Fig. 20D). Fig. 20 D zeigt ein besonders langes Ausführungsbeispiel eines Hinterohrelements 40 mit einer Gesamtlänge L von 150 mm.

### Bezugszeichenliste

| | |
|---|---|
| 10 | Verbindungselements |
| 20 | Bügel |
| 22 | Koppelsystem |
| 24 | Fixierungselement |
| 25 | Ankerelement |
| 26 | Kopplungselement |
| 30 | Anfang |
| 31 | Abschnitt |
| 32 | Wendepunkt |
| 33 | Abschnitt |
| 34 | Wendepunkt |
| 35 | Abschnitt |
| 36 | Wendepunkt |
| 37 | Endabschnitt |
| 38 | Ende |
| 40 | Hinterohrelement |
| 41 | Endstück |
| 42 | Halteelement |
| 43 | Endbereich |
| 44 | Ende |
| 45 | Mittelstück |
| 45i | Zweites Mittelstück |
| 46 | Öse |
| 46i | Zweite Öse |
| 48 | Verbindungsnut |
| 49 | Öffnung |
| 50 | Lumen |
| 60 | Kopfband |
| 62 | Befestigungselement |
| 63 | Endbereich |
| 64 | Ende |
| 70 | Beatmungsgerät |
| 80 | Leitung |
| 90 | Patienteninterface |
| 91 | Nasenkissen |
| 92 | Nasalprong |
| 93 | Schlauchhalterung |
| 95 | Kopf |
| 100 | System |
| B | Breite |
| D | Dicke |
| F | Zugkraft |
| J | Jochbeinregion |
| L | Länge |
| M1, M2 | Mittelpunkt |
| R1, R2 | Radius |
| S | Symmetrieebene |
| x | x-Achse |
| y | y-Achse |
| z | z-Achse |
| α1, α2 | Mittelpunktswinkel |
| β | Winkel |

## Patentansprüche

1. Verbindungselement (10) für ein Patienteninterface (90), wobei das Verbindungselement (10) zwei Bügel (20) zur Verbindung mit dem Patienteninterface (90) umfasst, wobei die Bügel (20) einen Anfang (30) und ein Ende (38) umfassen, wobei zwischen dem Anfang (30) und dem Ende (38) mindestens drei Wendepunkte (32, 34, 36) liegen und zwischen dem Anfang (30), den mindestens drei Wendepunkten (32, 34, 36) und dem Ende (38) mindestens vier Abschnitte (31, 33, 35, 37) liegen, wobei das Verbindungselement (10) ferner zwei separate Hinterohrelemente (40) zur Kopplung an jeweils einen der beiden Bügel (20) umfasst, wobei die Hinterohrelemente (40) in einem Anwendungszustand am und/oder hinter dem Ohr angeordnet sind, wobei benachbart zum Anfang (30) ein Koppelsystem (22) zur Kopplung des Bügels (20) an das Patienteninterface (90) eingerichtet und ausgebildet ist, wobei benachbart zum Ende (38) ein Endabschnitt (37) angeordnet ist, an den das jeweilige Hinterohrelement (40) koppelbar ist, wobei die Hinterohrelemente (40) jeweils mindestens ein Halteelement (42) umfassen, das jeweils an den Endabschnitt (37) eines der beiden Bügel (20) koppelbar ist, **dadurch gekennzeichnet, dass** die Halteelemente (42) als Hohlkörper mit einem Lumen ausgebildet sind, in das der Endabschnitt (37) des jeweiligen Bügels (20) zur Koppelung an mindestens einer Position platziert wird, wobei die Halteelemente (42) an beliebige Positionen des jeweiligen Endabschnitts (37) verschiebbar gekoppelt werden und derart eine Längenverstellung des Verbindungselements (10) gegeben ist.

2. Verbindungselement (10) nach Anspruch 1, **dadurch gekennzeichnet, dass** die Hinterohrelemente (40) reversibel und/oder irreversibel an die Bügel (20) koppelbar sind.

3. Verbindungselement (10) nach einem der vorangehenden Ansprüche, **dadurch gekennzeichnet, dass** die Bügel (20) zumindest aus einem versteifenden und zugleich flexiblen Material und/oder Materialverbund hergestellt sind ausgewählt aus der Gruppe Kunststoffe, Metalle, Silikone und Schaumstoffe, bevorzugt aus Polypropylen.

4. Verbindungselement (10) nach einem der vorangehenden Ansprüche, **dadurch gekennzeichnet, dass** die Bügel (20) eine Länge L und eine Breite B aufweisen, wobei das Verhältnis der Länge L zur Breite B mindestens 10:1, bevorzugt 20:1, besonders bevorzugt 25:1 oder mehr beträgt.

5. Verbindungselement (10) nach einem der vorangehenden Ansprüche, **dadurch gekennzeichnet, dass** die Hinterohrelemente (40) aus einem elastischen Material gefertigt sind ausgewählt aus der Gruppe Silikone, Thermoplastischen Elastomere, Neoprene und Textilien, bevorzugt aus Silikon.

6. Verbindungselement (10) nach einem der vorangehenden Ansprüche, **dadurch gekennzeichnet, dass** die Endabschnitte (37) mindestens ein Ankerelement (25) umfassen, um die Kopplung des Halteelements (40) an den Bügel (20) zu verankern.

7. Verbindungselement (10) nach einem der vorangehenden Ansprüche, **dadurch gekennzeichnet, dass** die Endabschnitte (37) alternativ oder zusätzlich ein oder mehrere Fixierungselemente (24) umfassen, um die Kopplung des Hinterohrelements (40) an den Bügeln (20) zu fixieren.

8. Verbindungselement (10) nach Anspruch 7, **dadurch gekennzeichnet, dass** die Fixierung der Hinterohrelemente (40) an den Bügeln (20) eine stufenweise Längenverstellung des Verbindungselements (10) zulässt.

9. Verbindungselement (10) nach einem der vorangehenden Ansprüche, **dadurch gekennzeichnet, dass** die Hinterohrelemente (40) eine Länge L und eine Breite B aufweisen, wobei das Verhältnis der Länge L zur Breite B des Hinterohrelements (40) mindestens 1:1, bevorzugt 5:1 oder mehr beträgt.

10. System (100) zur Beatmung und/oder Atemunterstützung mindestens umfassend ein Patienteninterface (90), ein Verbindungselement (10) zur Verbindung mit dem Patienteninterface (90), ein Beatmungsgerät (70), mindestens eine Leitung (80), wobei das Patienteninterface (90) mit dem Verbindungselement gekoppelt ist und über das Verbindungselement (10) mit dem Kopf (95) eines Patienten verbunden ist, wobei das Patienteninterface (90) und das Beatmungsgerät (70) über die Leitung (80) gasleitend miteinander verbunden sind, **dadurch gekennzeichnet, dass** das Verbindungselement (10) nach einem der vorhergehenden Ansprüche eingerichtet und ausgebildet ist.

## Claims

1. A connecting element (10) for a patient interface (90), wherein the connecting element (10) comprises two straps (20) for connecting to the patient interface (90), wherein the straps (20) comprise a beginning (30) and an end (38), wherein at least three inflection points (32, 34, 36) lie between the beginning (30) and the end (38) and at least four portions (31, 33, 35, 37) lie between the beginning (30), the at least three inflection points (32, 34, 36), and the end (38), wherein the connecting element (10) also comprises two separate behind-the-ear elements (40), each for coupling to one of the two straps (20), wherein the behind-the-ear elements (40) are arranged on and/or behind the ear in a use state, wherein a coupling system (22) adjacent to the beginning (30) is configured and designed to couple the strap (20) to the patient interface (90), wherein an end portion (37) is arranged adjacent to the end (38), to which end portion the respective behind-the-ear element (40) can be coupled, wherein the behind-the-ear elements (40) each comprise at least one retaining element (42), each of which can be coupled to the end portion (37) of one of the two straps (20), **characterized in that** the retaining elements (42) are designed as a hollow body with a lumen, into which the end portion (37) of the respective strap (20) is placed for coupling at at least one position, wherein the retaining elements (42) are coupled in a displaceable manner at any positions of the respective end portions (37) and the length of the connecting element (10) can thereby be adjusted.

2. The connecting element (10) according to claim 1, **characterized in that** the behind-the-ear elements (40) can be reversibly and/or irreversibly coupled to the straps (20).

3. The connecting element (10) according to one of the preceding claims, **characterized in that** the straps (20) are produced at least from a stiffening and simultaneously flexible material and/or composite material selected from the group of plastics, metals, silicones, and foams, preferably from polypropylene.

4. The connecting element (10) according to one of the preceding claims, **characterized in that** the straps (20) have a length L and a width B, wherein the ratio of the length L to the width B is at least 10:1, preferably 20:1, particularly preferably 25:1 or more.

5. The connecting element (10) according to one of the preceding claims, **characterized in that** the behind-the-ear elements (40) are manufactured from an elastic material selected from the group of silicones, thermoplastic elastomers, neoprenes, and textiles, preferably from silicone.

6. The connecting element (10) according to one of the preceding claims, **characterized in that** the end portions (37) comprise at least one anchor element (25) in order to anchor the coupling of the retaining element (40) to the straps (20).

7. The connecting element (10) according to one of the preceding claims, **characterized in that** the end portions (37) alternatively or additionally comprise one or more fixing elements (24) in order to fix the coupling of the behind-the-ear elements (40) to the straps (20).

8. The connecting element (10) according to claim 7, **characterized in that** the fixing of the behind-the-ear elements (40) to the straps (20) allows the length of the connecting element (10) to be adjusted in steps.

9. The connecting element (10) according to one of the preceding claims, **characterized in that** the behind-the-ear elements (40) have a length L and a width B, wherein the ratio of the length L to the width B of the behind-the-ear elements (40) is at least 1:1, preferably 5:1 or more.

10. A system (100) for ventilation and/or respiratory support at least comprising a patient interface (90), a connecting element (10) for connecting to the patient interface (90), a ventilator (70), at least one line (80), wherein the patient interface (90) is coupled to the connecting element and is connected to the head (95) of a patient via the connecting element (10), wherein the patient interface (90) and the ventilator (70) are connected to each other in a gas-conducting manner via the line (80), **characterized in that** the connecting element (10) is configured and designed according to one of the preceding claims.

## Revendications

1. Élément de connexion (10) pour une interface patient (90), dans lequel l'élément de connexion (10) comporte deux branches (20) pour la connexion à l'interface patient (90), dans lequel les branches (20) comportent un début (30) et une fin (38), dans lequel au moins trois points d'inflexion (32, 34, 36) se situent entre le début (30) et la fin (38) et au moins quatre sections (31, 33, 35, 37) se situent entre le début (30), les au moins trois points d'inflexion (32, 34, 36) et la fin (38), dans lequel l'élément de connexion (10) comporte en outre deux éléments de tube arrière (40) séparés, destinés à être accouplés respectivement à l'une des deux branches (20), dans lequel les éléments de tube arrière (40) sont disposés sur et/ou derrière l'oreille dans un état d'utilisation, dans lequel un système d'accouplement (22) adjacent au début (30) est configuré et réalisé pour l'accouplement de la branche (20) à l'interface patient (90), dans lequel une section de fin (37), à laquelle l'élément de tube arrière (40) respectif peut être accouplé, est disposée à côté de la fin (38), dans lequel les éléments de tube arrière (40) comportent respectivement au moins un élément de maintien (42) apte à être accouplé respectivement à la section de fin (37) de l'une des deux branches (20), **caractérisé en ce que** les éléments de maintien (42) sont réalisés comme des corps creux avec un lumen, dans lequel est placée la section de fin (37) de la branche (20) respective pour l'accouplement à au moins une position, dans lequel les éléments de maintien (42) sont accouplés de façon déplaçable à des positions au choix de la section de fin (37) respective, fournissant ainsi un réglage de la longueur de l'élément de connexion (10).

2. Élément de connexion (10) selon la revendication 1, **caractérisé en ce que** les éléments de tube arrière (40) peuvent être accouplés de façon réversible et/ou irréversible aux branches (20).

3. Élément de connexion (10) selon l'une des revendications précédentes, **caractérisé en ce que** les branches (20) sont fabriquées au moins à partir d'un matériau et/ou d'un matériau composite à la fois rigidifiant et souple, sélectionné parmi le groupe comprenant des matières plastiques, des métaux, des silicones et des mousses, de préférence à partir de polypropylène.

4. Élément de connexion (10) selon l'une des revendications précédentes, **caractérisé en ce que** les branches (20) présentent une longueur L et une largeur B, dans lequel le rapport de la longueur L à la largeur B est au moins de 10:1, préférentiellement de 20:1, particulièrement préférentiellement de 25:1.

5. Élément de connexion (10) selon l'une des revendications précédentes, **caractérisé en ce que** les éléments de tube arrière (40) sont fabriqués à partir d'un matériau élastomère sélectionné parmi le groupe comprenant des silicones, des élastomères thermoplastiques, des néoprènes et des textiles, préférentiellement à partir de silicone.

6. Élément de connexion (10) selon l'une des revendications précédentes, **caractérisé en ce que** les sections de fin (37) comportent au moins un élément d'ancrage (25) permettant d'ancrer l'accouplement de l'élément de maintien (42) à la branche (20).

7. Élément de connexion (10) selon l'une des revendications précédentes, **caractérisé en ce que** les sections de fin (37) comportent alternativement ou également un ou plusieurs éléments de fixation (24) permettant de fixer l'accouplement de l'élément de tube arrière (40) aux branches (20).

8. Élément de connexion (10) selon la revendication 7, **caractérisé en ce que** la fixation de l'élément de tube arrière (40) aux branches (20) permet un réglage progressif de la longueur de l'élément de connexion (10).

9. Élément de connexion (10) selon l'une des revendications précédentes, **caractérisé en ce que** les éléments de tube arrière (40) présentent une longueur L et une largeur B, dans lequel le rapport de la longueur L à la largeur B de l'élément de tube arrière (40) est au moins de 1:1, préférentiellement de 5:1 ou plus.

10. Système (100) de ventilation et/ou d'assistance respiratoire comportant au moins une interface patient (90), un élément de connexion (10) destiné à la connexion avec l'interface patient (90), un appareil respiratoire (70), au moins une conduite (80), dans lequel l'interface patient (90) est accouplée à l'élément de connexion et reliée à la tête (95) d'un patient par le biais de l'élément de connexion (10), dans lequel l'interface patient (90) et l'appareil respiratoire (70) sont reliés entre eux par le biais de la conduite (80) de manière à conduire un gaz, **caractérisé en ce que** l'élément de connexion (10) est configuré et réalisé selon l'une des revendications précédentes.
